# EUROPEAN PATENT SPECIFICATION

(11) **EP 4 452 103 B1**
(45) Date of publication and mention of the grant of the patent: **29.10.2025**
(21) Application number: 22844127.5
(22) Date of filing: 23.12.2022
(51) Int. Cl.: A61B 17/66, A61B 17/62, A61B 17/17, A61B 17/84, A61B 17/86, A61B 17/88

(54) **ORTHOPEDIC CABLE BONE TRANSPORT DEVICE AND BONE TRANSPORT SYSTEM COMPRISING SAID DEVICE**
KNOCHENTRANSPORTVORRICHTUNG MIT ORTHOPÄDISCHE KABEL UND KNOCHENTRANSPORTSYSTEM MIT DIESER VORRICHTUNG
DISPOSITIF DE TRANSPORT OSSEUX PAR CÂBLE ORTHOPÉDIQUE ET SYSTÈME DE TRANSPORT OSSEUX COMPRENANT LEDIT DISPOSITIF

(30) Priority: 23.12.2021 US 202117560789
(43) Date of publication of application: 30.10.2024
(73) Proprietor: Orthofix S.r.l., 37012 Bussolengo (VR) (IT); TEXAS SCOTTISH RITE HOSPITAL FOR CHILDREN, Dallas, TX 75219 (US)
(72) Inventor: VENTURINI, Daniele, 37064 Povegliano Veronese (VR) (IT); OTTOBONI, Andrea, 45020 Giacciano con Baruchella (RO) (IT); LUPATINI, Michael, 25015 San Martino della Battaglia (BS) (IT); ROSS, John D., Ovilla, Texas 75154-5832 (US); SAMCHUKOV, Mikhail L., Coppel, Texas 75019 (US); CHERKASHIN, Alexander M., Flower Mound, Texas 75022 (US); STANDEFER, Karen D., Flower Mound, Texas 75022 (US)
(74) Representative: Botti & Ferrari S.p.A.
(86) International application number: PCT/EP2022/087716
(87) International publication number: WO 2023/118562

(56) References cited:
- EP-A1- 0 428 985
- EP-A1- 2 772 215
- EP-A1- 3 560 446
- WO-A1-2011/097672
- WO-A1-2016/174420
- WO-A1-2019/168817
- WO-A1-2022/240692
- WO-A2-2009/109371
- GB-A- 2 207 055
- US-A- 4 570 618
- US-A- 4 622 960
- US-A- 6 030 162
- US-A- 6 033 412
- US-A1- 2004 210 227
- US-A1- 2013 190 830
- US-A1- 2019 133 654
- US-B1- 6 491 714
- ROSTEIUS THOMAS ET AL: "Ilizarov bone transport using an intramedullary cable transportation system in the treatment of tibial bone defects", vol. 52, no. 6, 1 June 2021 (2021-06-01), GB, pages 1606 - 1613, XP055919129, ISSN: 0020-1383, Retrieved from the Internet <URL:https://www.sciencedirect.com/science/article/pii/S0020138320310615/pdfft?md5=022a1bf11fe90183e305e1e66ddda8eb&pid=1-s2.0-S0020138320310615-main.pdf> [retrieved on 20220509], DOI: 10.1016/j.injury.2020.12.028

## Description

### CROSS-REFERENCE TO RELATED APPLICATIONS

This application is claiming priority from the US patent application No. US 17/560,789 filed on Dec. 23, 2021.

### TECHNICAL FIELD

The present invention is generally directed to a cable pulling device and an associated orthopedic bone transport system.

The invention therefore has a useful application in the sector of orthopedics, in particular in bone defects treatment.

### BACKGROUND OF THE DISCLOSURE

External fixation systems are used in a variety of surgical procedures including fracture reduction, limb lengthening, and deformity correction, as well as treatment of non-unions, mal-unions, and bone defects. For bone defects treatment, a rigid framework comprising of upper (proximal) and lower (distal) external circular supports is placed externally around an affected limb and attached to associated upper (proximal) and lower (distal) bone segments using wires and/or pins. The proximal and distal external supports of the rigid framework are interconnected by threaded or telescopic rods stabilizing aligned spatial positioning of the distal bone segment relative to the proximal bone segment. One of those bone segments (e.g., proximal) is then divided into two bone segments (e.g., via osteotomy), thereby producing a third (intercalary) bone segment also called a transport bone segment that is gradually transported through the bone defect area creating newly formed bone tissues in the path of that transportation.

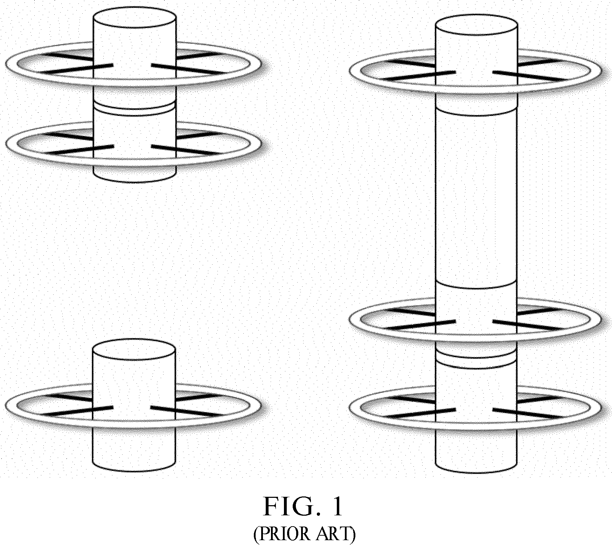

It is beneficial to have a stable fixation of the transport bone segment for controlled movement of that transport bone segment through the area of bone defect with minimal penetration of surrounded soft tissues by fixation wires and/or pins. For example, it may be beneficial to secure the transport segment to a separate intercalary external support (e.g., transport external support) via transverse wires and/or pins and gradually move that intercalary support with attached transport bone segment along the rods interconnecting the proximal and distal external supports (Fig. 1, transverse-wires bone transport). Although such structure provides sufficient stability of the transport bone segment fixation, it typically produces multiple long longitudinal cuts through the surrounding soft tissues by wires and/or pins potentially resulting in pain, infection, and other complications.

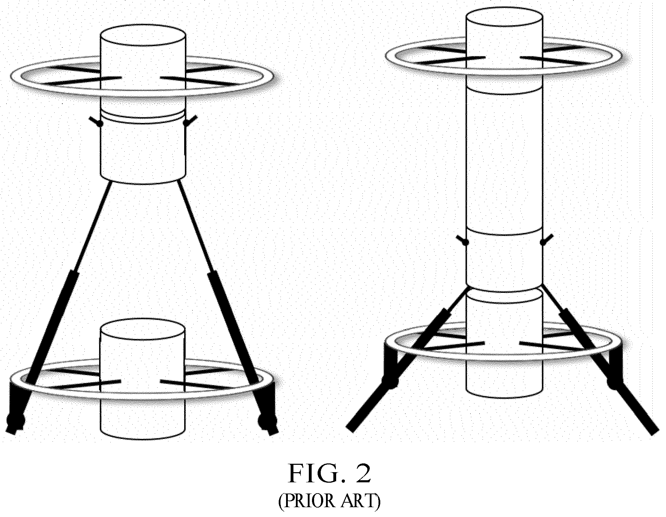

Stabilization of transport bone segment via two wires with stoppers obliquely inserted into transport bone segment have been sporadically used for bone transport. This method of bone defect treatment is known as the Ilizarov bone transport (Fig.2, oblique-wires bone transport). Utilization of this method is significantly reduced recently due to several limitations including difficulties in insertion and removal of obliquely oriented wires into/from the transport bone segment, limited distance of transport bone segment transportation through the bone defect area, pressure of obliquely oriented wires on the surrounding soft tissues at the exit points, and potential longitudinal misalignment of the transport bone segment with the residual proximal and distal segments at the end of bone transport.

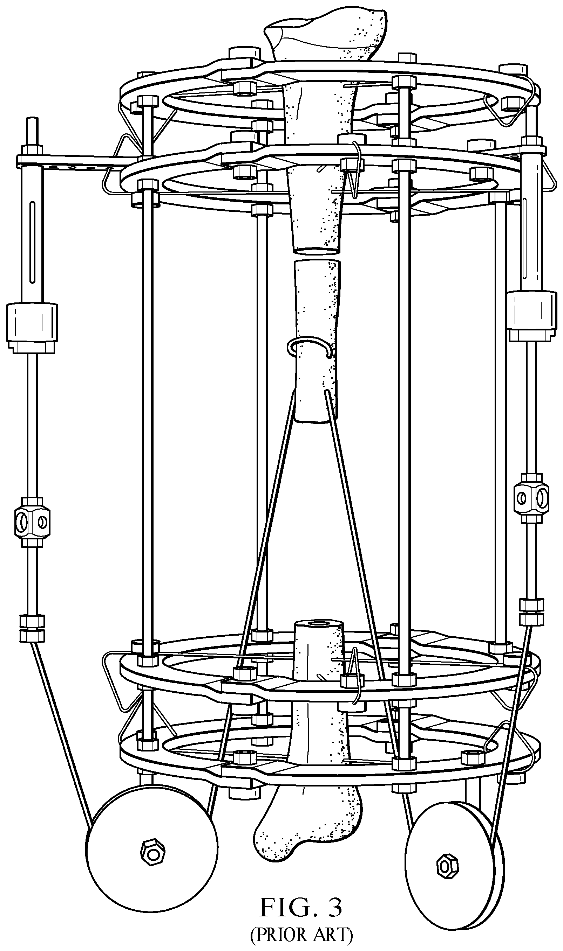

Stabilization of transport bone segment via cable have also been traditionally used for bone transport. This method of bone defect treatment is known as the Weber bone transport (Fig. 3, Weber cable bone transport with rollers and gradual cable pulling rods). In those cases, a thin 1.8 mm diameter stainless-steel cable is inserted through the end of the transport bone segment, placed longitudinally parallel to the path of the transport bone segment movement, exiting out at the level of docking between the transport bone segment and one of the residual bone segments (e.g., distal bone segment). The ends of the cable are then placed into grooves of the special plastic rollers redirecting the ends of the cable towards proximal external support and attached to threaded or telescopic rods providing gradual pull of the cable with attached transport bone segment through the area of bone defect. An enhanced version of the Weber cable bone transport, known as the Balanced Cable Bone transport, was recently introduced by Dr. Stephen Quinnan.

Although cable bone transport significantly reduced soft tissue related complication, the process of transport bone segment transportation using path redirection rollers and threaded rods for gradual adjustment located far away from the rollers is very complicated. In addition, rollers and pulling rods occupy space on the external supports limiting places for other connecting and wire/pin fixation elements. Finally, the cable ends located between the proximal and distal external supports are unprotected and can be damaged from the outside failing to provide sufficient stability of transport bone segment fixation.

Document EP 3 560 446 A1 discloses a cable pulling device according to the prior art.

Article of Rosteius Thomas et al. with title "Ilizarov bone transport using an intramedullary cable transportation system in the treatment of tibial bone defects" (INJURY, vol. 52, no. 6, 1 June 2021, pages 1606-1613) discloses an orthopedic bone transport system according to the prior art.

Documents US 2019/133654 A1 and EP 2772 215 A1 disclose medical cables according to the prior art.

Document WO 2016/174420 A1 discloses a medical instrument according to the prior art;
Documents US 6,491,714 B1 and WO 2009/109371 A2 disclose drill guides according to the prior art.

Document US 6,030,162 A discloses a bone screw according to the prior art.

The technical problem underlying the present invention is therefore that of devising a cable bone transport systems that solves at least some of the drawbacks of the prior art, and in particular which provides sufficient stability of the transport bone segment, minimizes number of external components between the proximal and distal external supports, and simplifies transportation of the intercalary transport bone segment during the bone transport.

### SUMMARY OF THE INVENTION

The idea for a solution forming basis of the present invention is that of proposing a device, which uses a mechanical transmission driving by small increments a reel that pulls the cable to which the transport bone segment is attached.

By means of such a device, a new bone transport technique can be implemented, wherein the cable enters at a first the intercalary transport bone segment at its resected end and is attached thereto by means of an external loop, passing through at least one tangential hole made on the transport bone segment into its intermedullary canal and bone defect area following the bone axis and the pass of the movement of the transport bone segment, enters the residual bone segment (proximal or distal) from the intermedullary canal and, after orthogonal redirection around a fulcrum pin or screw, exits that residual bone segment, surrounding soft tissues and skin to be attached to the cable pulling device.

The aforementioned technical problem is therefore solved by a cable pulling device for an orthopedic bone transport system, comprising: a main body configured to be secured to an external fixation frame solidly attached to at least one fixed bone segment; a reel rotatable about said main body, for winding up a cable to be secured to a transport bone segment, thus moving said transport bone segment towards said fixed bone segment; a transmission mechanism driving the reel in order to wind up the cable.

The reel and the related transmission mechanism therefore define a winch acting on the cable connected to the transport bone segment: by driving the winch, the cable is pulled, and the transport bone segment is approached to the fixed bone segment.

Preferably, the reel is only rotatable in a single direction, so that the tension on the cable cannot be released by winding it out.

In a preferred embodiment, the main body is a case which houses at least partially the transmission and the reel.

Preferably, the transmission mechanism is a worm gear mechanism. The worm gear mechanism may define an irreversible kinematic pair, meaning that the motion can be transmitted from an input worm to an output worm gear but not from the worm gear to the worm.

Preferably, the worm gear mechanism comprises: a worm rotatably fixed to the main body; and a worm gear rotatably fixed to the main body and engaging with said worm, wherein said reel integrally rotates with said worm gear.

Preferably, wherein the worm gear is coaxially provided with the reel.

The cable pulling device may further comprise a knob to transmit a rotating motion to said worm. In particular, the knob can be periodically rotated by a user - preferably a healthcare practitioner - to displace the transport bone segment toward the fixed bone segment by a predefined increment.

Preferably, the knob is mounted on the same shaft of the worm. The knob can be free to axially slide on said shaft between an extracted position and a retracted position.

Preferably, the knob has a locking mechanism that locks the rotation of the knob with respect to the main body at given locking angular positions of the knob. These locking angular positions may correspond to fixed angular increments that translate into predefined incremental displacements of the transport bone segment. In this manner, the locking mechanism guides the user into applying the right amount of displacement to the transport bone segment.

The fixed angular increments may correspond to one fourth of a turn (i.e. 90°), one sixth of a turn (i.e. 60°), one eighth of a turn (i.e. 45°), or may correspond to yet another increment.

Preferably, the predefined linear increment in the cable displacement is comprised between 0.25 and 0.5 mm. In the specific case of a knob angular increment of one sixth of a turn, this is given by the formula d = (D·π)/6·n, wherein D is the diameter of the reel and n is the transmission ratio of the transmission mechanism. Obviously, the formula should be modified for the alternative angular increments given above.

Preferably, the diameter D of the reel is between 25 mm and 50 mm, most preferably it is equal to about 30 mm (28.6 mm), resulting in 90 mm of circumference of the reel.

In a preferred embodiment, the locking mechanism comprises a regular polygon boss and a mating recess, respectively provided on the knob and on the main body, or the other way around. The rotation of the worm is prevented when the regular polygon boss is pressed into the recess, which happens because of the action of an elastic element biasing the knob towards the main body. Therefore, to rotate the knob one has to extract it from the position in which the boss seats in the recess, and then the knob freely rotates until the next alignment position between boss and recess is reached.

A hexagonal boss and recess can be used to achieve a fixed angular increment of one sixth of a turn; correspondingly, squared or octahedral bosses and recesses could be employed for fixed angular increments of one fourth or one eighth of a turn; other polygonal shapes may be used for other angular increments.

As an alternative to manual activation, the cable pulling device might be activated by means of a motor. In particular, the motor can be automatically activated, for instance at preset time intervals.

The device can therefore comprise a motor coupled with the worm to drive the rotation of the worm and a controller for controlling the motor, wherein the controller is configured to control an activation frequency of the motor and a rotation angle of the worm driven by the motor for each activation of the motor.

Preferably, the cable pulling device comprises a pawl acting as a ratchet device, i.e. configured to prevent a reverse rotation of the worm gear under a force acting on the cable.

Preferably, the reel is provided with a radial hole through which the cable passes, and the cable is clamped by an end of a threaded shaft integral with the worm gear.

Preferably, the reel comprises a spiral groove on its external surfaces to receive the cable as it winds up, preventing overlapping.

In a possible embodiment, the cable pulling device comprises a force sensor for sensing a tension force acting on the cable. The force sensor may be directly or indirectly coupled to the cable. In other words, it may be placed on the cable itself or on other elements interacting with the cable, for instance on the reel.

The technical problem is also solved by a bone transport system, comprising: an external fixation frame configured to be attached to at least one fixed bone segment; and at least one cable pulling device as previously described, solidly attached to the external fixation frame; and means for redirecting the cable configured to be attached to the fixed bone segment and to redirect the cable departing from the cable pulling devices towards a bone transport segment.

Preferably, the bone transport system comprises two cable pulling devices solidly attached to the external fixation frame in diametrically opposite positions, wherein the two cable pulling devices hold the opposite ends of a same cable.

Preferably, the cable is configured to depart from the cable pulling devices, enter the fixed bone segment via lateral holes, redirect in an axial direction of the bone at the means for redirecting the cable, enter the bone transport segment, exit the same bone transport segment via at least one lateral hole, loop around said bone transport segment.

In an aspect of the present disclosure, a method of installing a bone transport system includes drilling a first bore through a transport bone segment, securing a cable to the transport bone segment by routing the cable through the first bore, drilling a second bore in a fixed bone segment, routing the cable through the second bore, and winding the cable with at least one cable pulling device to tension the cable between the transport bone segment and the fixed bone segment to urge the transport bone segment toward the fixed bone segment.

In some examples, a method may further include securing the at least one cable pulling device to a first external fixation ring. The at least one cable pulling device may include a first cable pulling device. The first cable pulling device may be secured to the first external fixation ring with a first bolt. A method may also include securing the first cable pulling device to a second external fixation ring with a second bolt. The first external fixation ring may be secured to the fixed bone segment and the second external fixation ring may be secured to a second fixed bone segment disposed on an opposite side of the transport bone segment from the fixed bone segment.

In some examples, the at least one cable pulling device may include a second cable pulling device. The second cable pulling device may be secured to a portion of the first external fixation ring diametrically opposed from the first cable pulling device.

In some examples, routing the cable through the first bore may include forming a loop in the cable extending around an external surface of the transport bone segment from a first end of the bore to a second end of the bore such that a first end of the cable and a second end of the cable each extend from an intermedullary canal of the transport bone segment.

In some examples, routing the cable through the second bore may include passing a first end of the cable into an intermedullary canal of the fixed bone segment and out of the fixed bone segment through a first end of the second bore and passing a second end of the cable into the intermedullary canal of the fixed bone segment and out of the fixed bone segment through a second end of the second bore. The first bore may be oriented in a first direction and the second bore may be oriented parallel to the first bore. In some examples, a method may further include drilling a third bore through the fixed bone segment. The third bore may be oriented transverse to the first direction. A balance screw may be positioned in the third bore.

In some examples, a first portion of the cable may extend from a medial side of an intermedullary canal of the transport bone segment, into a medial side of an intermedullary canal of the fixed bone segment, and may exit the intermedullary canal of the fixed bone segment through a lateral side of the intermedullary canal of the fixed bone segment. A second portion of the cable may extend from the lateral side of the intermedullary canal of the transport bone segment, into the lateral side of the intermedullary canal of the fixed bone segment, and may exit the intermedullary canal of the fixed bone segment through medial side of the intermedullary canal of the fixed bone segment. The first and second portions of the cable may cross in a medial-lateral direction on a side of the balance screw opposite the transport bone segment.

In an aspect of the present disclosure, a cable pulling device for an orthopedic bone transport system may include a main body configured to be secured to an external fixation frame and a reel mounted to the main body. The reel may be configured to receive a portion of a cable. The cable pulling device may further include a driver configured to rotate the reel to wind the cable around the reel.

In some examples, the driver may include a worm gear secured to the reel and a worm screw configured to rotate the worm gear. The worm gear and the reel may be formed monolithically as a single component. The driver may further include a knob secured to the worm screw. The knob may be configured for manual rotation to wind the cable around the reel. A blind bore may extend into the reel. The blind bore may be configured to threadingly receive a set screw. A radial hole extends transverse to the blind bore and intersects the blind bore, the radial hole configured to receive a portion of the cable. The reel may include a helical groove configured to guide the cable such that adjacent coils formed in the cable around the reel are positioned along a rotation axis of the reel.

In some examples, the main body may include at least one slot configured to receive a portion of a fastener to secure the main body to the external fixation frame. The at least one slot may include a first slot formed within a first end of the main body and a second slot formed within a second end of the main body. In some examples, the main body may include at least one detachable mounting bracket configured to mount the cable pulling device to the external fixation frame.

In some examples, the main body includes a first window extending from an external surface to an internal cavity. The reel may be positioned within the cavity. The first window may be configured to receive the cable. The first window may include a horizontal portion oriented parallel to a rotation axis of the reel. The first window may include a vertical portion. The horizontal portion and the vertical portion may form an L-shape. The main body may further include a second window disposed through a wall of the main body opposite the first window. The reel may be enclosed in the cavity such that the reel is exposed only through the first window and the second window.

In another aspect of the present disclosure, a medical cable may include a cable formed of a plurality of strands and a lead wire extending from an end of the cable. The lead wire may have a smaller diameter than the cable and may be more flexible than the cable. The lead wire may be secured to the cable by an external sleeve crimped over the cable and lead wire or by an ultraviolet light-cured resin.

In another aspect of the present disclosure, a medical cable may include a first segment of cable having a first property and a second segment of cable having a second property. The second property being visibly distinguishable from the first property.

In some examples, the first property includes a first surface finish and the second property includes a second surface finish. The first surface finish may be smooth or glossy and the second surface finish may be rough or dull. In some examples, the first property includes a first color and the second property includes a second color. In some examples, the first property includes a first color of a first lead wire secured to a first end formed in the first segment of cable and the second property includes a second color of a second lead wire secured to a second end formed in the second segment of cable. In some examples, the first segment of cable and the second segment of cable may be equal. The first segment of cable may be a first half of the medical cable and the second segment of cable may be a second half of the medical cable.

In an aspect of the present disclosure, a medical instrument includes a shaft and a clip disposed adjacent to a first end of the shaft. The clip may extend away from the first end. A passage may be formed between an external surface of the shaft and the clip.

In some examples, the shaft is flexible. The clip may be flexible and configured to resiliently bend away from the shaft and trap a cable or wire within the passage. The clip may include an angled surface at an end of the clip. The angled surface may be configured to engage a cable or wire and to bend away from the shaft in response to a force exerted on the angled surface by a cable or wire as the shaft is moved in a direction transverse to a longitudinal axis of the cable or wire as the cable or wire slides along the shaft. The first end of the shaft may have an atraumatic tip. The shaft may include a first diameter along a majority of the shaft and a second diameter, smaller than the first diameter, along at least a portion of the shaft on which the clip is disposed.

In another aspect of the present disclosure, a drill guide includes a shaft, a first handle, and a second handle. A first end portion of the shaft may be configured to be received in an intermedullary canal of a bone. The first handle may include a first drill bore aligned with the first end portion of the shaft. The second handle may include a second drill bore aligned with the first end portion of the shaft. At least one of the first handle and the second handle may be configured to swivel about a longitudinal axis of the shaft.

In some examples, a grip portion of the first handle may extend parallel to the longitudinal axis of the shaft and may be secured to the shaft by a first bridge. A grip portion of the second handle may extend parallel to the longitudinal axis of the shaft and may be secured to the shaft by a second bridge. The grip portion of the first handle may extend from the first bridge in a direction in which the shaft extends from the first bridge. The grip portion of the second handle may extend from the second bridge in a direction in which the shaft extends from the second bridge.

In some examples, the first handle may be configured to swivel about the longitudinal axis of the shaft and the second handle may be configured to remain fixed in relation to the shaft. In some examples, a guide surface may extend radially outward from the longitudinal axis of the shaft. The guide surface may have a radial dimension configured to prevent the guide surface from entering the intermedullary canal. In some examples, the drill guide includes at least one spacer washer configured to be positioned around the shaft. The guide surface may be formed on the at least one spacer washer.

In some examples, the first drill bore may be offset from the second drill bore along the longitudinal axis of the shaft.

In some examples, the drill guide may include a stopper configured to limit rotation of the first handle with respect to the second handle to 90° such that the first drill bore may be positioned orthogonally with respect to the second drill bore.

In some examples, the first end portion of the shaft may include at least one slot or aperture configured to remain aligned with the first drill bore or the second drill bore as the first handle or the second handle is rotated about the longitudinal axis. At least a portion of the first end portion of the shaft may include diametrically opposed grooves configured to receive a respective first portion and second portion of a cable positioned in the intermedullary canal.

In an aspect of the present disclosure, a bicortical bone screw may include a first threaded region, a second threaded region, and an intermediate region disposed between the first threaded region and the second threaded region. The intermediate region may include a first cable guide and a second cable guide.

In some examples, the first cable guide may include a first roller configured to rotate about a longitudinal axis of the bone screw and the second cable guide may include a second roller configured to rotate about the longitudinal axis of the bone screw.

In some examples, the first cable guide may include a first groove formed transverse to a longitudinal axis of the bone screw and the second cable guide may include a second groove formed transverse to the longitudinal axis of the bone screw.

In some examples, the first cable guide may include a first helical groove and the second cable guide may include a second helical groove parallel to the first helical groove. The first and second helical grooves may be formed between threads of the bone screw having a smaller diameter than threads of the first threaded region and threads of the second threaded region.

It is to be understood that both the foregoing general description and the following drawings and detailed description are exemplary and explanatory in nature and are intended to provide an understanding of the present disclosure without limiting the scope of the present disclosure. In that regard, additional aspects, features, and advantages of the present disclosure will be apparent to one skilled in the art from the following. One or more features of any implementation or aspect may be combinable with one or more features of other implementation or aspect.

Features and advantages of the present invention will be disclosed with reference to the enclosed drawings relating to an indicative and a non-limiting implementation example.

### BRIEF DESCRIPTION OF THE DRAWINGS

The accompanying drawings illustrate implementations of the systems, devices, and methods disclosed herein and together with the description, serve to explain the principles of the present disclosure.
FIG. 1 shows an example of a transverse-wires bone transport technique according to the prior art.
FIG. 2 shows an example of an oblique-wires bone transport technique according to the prior art.
FIG.3 shows an example of a Weber cable bone transport technique, with rollers and gradual cable pulling rods according to the prior art.
FIG. 4 shows a perspective view of a cable pulling device for bone transport according to an embodiment of the present invention.
FIG. 5 shows an exploded view of the cable pulling device of FIG. 4.
FIG. 6 shows a perspective view of a detail of a worm gear mechanism in the cable pulling device of FIG. 4.
FIG. 7 shows a perspective view of a detail of a worm fitted on its corresponding shaft in the worm gear mechanism of FIG. 7.
FIG. 8 shows an exploded view of a detail of a worm gear and reel in the worm gear mechanism of FIG. 7.
FIG. 9 shows a perspective view of a detail of a main body in the form of a case of the cable pulling device of FIG. 4.
FIG. 10 shows an exploded view of a detail of the case and knob of the cable pulling device of FIG. 4.
FIG. 11 is a cross-sectional view of the pulling device of FIG. 4 which emphasizes the ratchet mechanism acting on the worm screw.
FIG. 12 shows a perspective view of a bone transport system according to the present invention.
FIG. 13 shows a cross-sectional view of a reel and worm gear of a cable pulling device as shown in FIG. 14A.
FIG. 14A shows a perspective view of a cable pulling device according to the present disclosure.
FIG. 14B shows a perspective view of a cable pulling device according to the present disclosure.
FIG. 15 shows a front view of the cable pulling device of FIG. 14A.
FIG. 16 shows a rear view of the cable pulling device of FIG. 4.
FIG. 17 shows a rear view of the cable pulling device of FIG. 14A.
FIG. 18 shows a rear view of the cable pulling device of FIG. 14A illustrating internal components.
FIG. 19 shows a perspective view of a bone transport system according to the present disclosure.
FIG. 20 is a flowchart of an example method of installing a bone transport system according to the present disclosure.
FIG. 21 shows a known method of attaching a lead wire to a cable.
FIG. 22 shows a known cable with an attached lead wire.
FIGS. 23-24 and 26-27 illustrate various stages of preparing a transport bone segment according to the method of FIG. 20.
FIG. 25 shows a hook tool according to the present disclosure.
FIGS. 28-31 and 35-36 show various stages of preparing a fixed bone segment according to the method of FIG. 20.
FIGS. 32-33 show examples of a cable balance screw according to the present disclosure.
FIG. 34A shows a drill guide according to the present disclosure.
FIG. 34B shows a cross-section of a portion of the drill guide of FIG. 34A disposed within a bone segment during use.

These Figures will be better understood by reference to the following Detailed Description.

### DETAILED DESCRIPTION

In the following detailed description, reference is made to the accompanying drawings that form a part hereof and in which is shown, by way of illustration, specific embodiments. In the drawings, like numerals describe substantially similar components throughout the several views. Other embodiments may be disclosed, and structural changes may be made without departing from the scope of the present disclosure. The following detailed description is, therefore, not to be taken in a limiting sense.

The present disclosure relates to a segmental bone transport system 1 which adopts an innovative technique based on the pulling of a cable 9 by a reel 8.

The bone transport system 1 is designed to apply a translation force to a transport bone segment 10 by decreasing the length of a flexible cable 9 that is attached to that transport bone segment 10. To do so, it adopts a concept which is similar to that used to tune stringed instruments. One end of the cable 9 is secured to a reel 8 onto which it is wound via a worm gear mechanism 4.

In the case of a musical string, the other end of the string is fixed and not allowed to translate. As the tuning mechanism is turned, the tension of the string is changed which also changes it resonate frequency. The worm gear mechanism ensures that the tension will be maintained when the mechanism is no longer being turned. The worm can turn the worm gear to tighten the cable, but the worm gear cannot turn the worm to release the tension on the cable.

In the bone transport system 1 according to the current invention, tensioning the cable 9 by shortening it causes the bone transport segment 10 to move. The distance that the bone transport segment 10 can travel is a function of how much cable 9 can be wind around the reel 8.

Bone segment transport is performed in steps where the distance of transport for each step is preferably 0.25 to 0.50 mm performed two to four times a day. In most cases, a total of 1.0 mm of transport distance per day is desired.

The preferred embodiment of the invention herein described is configured to shorten the cable 0.25 mm for each one sixth turn of an activation knob 12.

To ensure that the knob 12 is not accidentally turned between adjustments, a spring-loaded locking mechanism, which will be better described in the following, secures the knob at each 60° turn increment.

Like the instrument tuning key, the worm gear mechanism 4 is used to ensure that the cable does not reverse direction under load. The reduction ratio of the worm gear mechanism 4 and the diameter of the reel 8 determine the amount of length change of the cable for a given activation increment of the knob 12.

An appropriate reel diameter can be derived with the following equation: Reel Diameter = (Increments per revolution) (increment distance per activation) (gear reduction)/ π. The ideal reel diameter for this device was determined to be 28.648 mm. This diameter was found to be large enough to allow the relatively stiff cable 9 to wind flat on it but not so large to be bulky and require more than a single set of reduction gears.

To ensure a uniform rate of transport, it is important to ensure that the cable 9 does not wind on top of itself. To ensure that this does not happen, the reel circumference contains a spiral groove 34 to guide the cable 9 and prevent it from overlapping.

The transport distance for each revolution of the reel is a function of the reel circumference. For this device which has a reel diameter of 28.648mm, the circumference would be π·28.648 mm = 90 mm. Therefore, each full turn of the reel 8 would shorten the cable by 90 mm and move the bone transport segment 10 by the same amount of distance.

The cable 9 is secured to the reel by passing through an oblique radial hole 32 and then clamped in place by an end of an axial screw 26. Excess cable is cut off prior to starting the transport process.

The worm gear mechanism 4 with the reel 8 are comprised in a cable pulling device 1 and substantially contained in a main body 2 or case.

The main body 2 is attached by means of a bolt 14 to a distal ring 38 of an external fixation frame. The bolt 14, which has an interposed washer 45, allows the position distance of the device with respect to the skin to be adjusted when the device is attached to said ring 38.

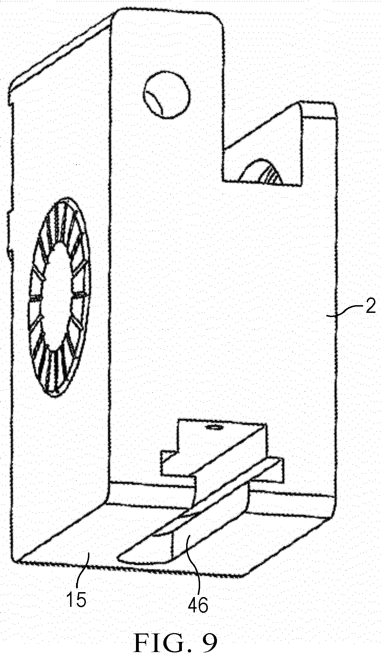

As visible in Figure 9, The main body 2 has an elongated slot 46, which extends in a transverse direction and receives the mounting screw 14 and the washer 45.

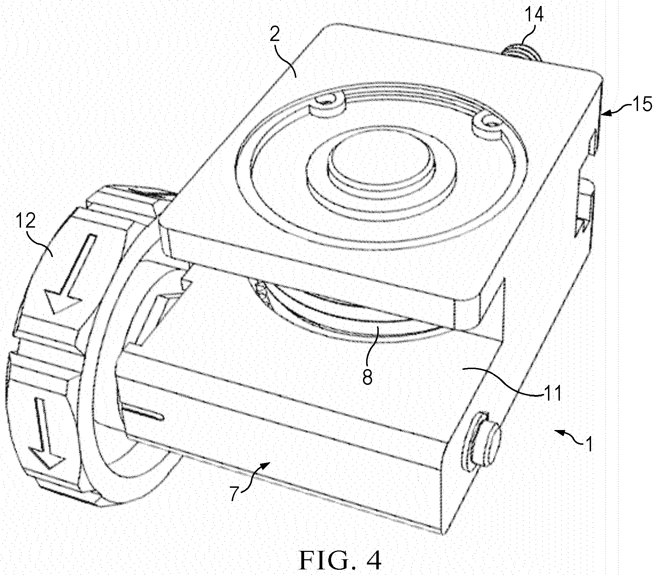

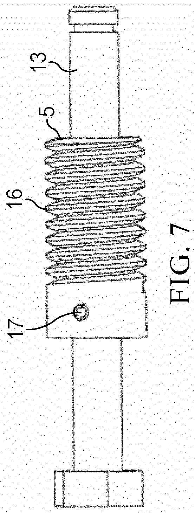

The cable pulling device 1 employed in the bone transport system is described below in further detail, with reference to enclosed FIGS. 4-11.

As visible in Figure 4, the cable pulling device 1 comprises the main body 2 and a worm gear mechanism 4 arranged in the main body 2.

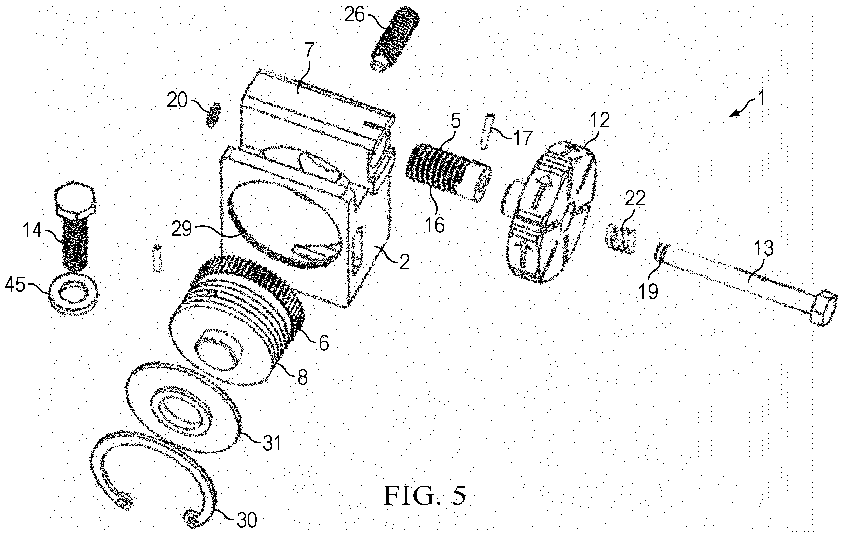

As visible in Figure 5, the worm gear mechanism 4 comprises a worm 5 and a worm gear 6. The worm 5 is rotatably fixed within an end portion 7 of the main body 2, wherein the worm gear 6 is rotatably fixed in a central portion of the main body 2 and engages with the worm 5.

The worm gear 6 is coaxially coupled with a reel 8. When the cable pulling device 1 is in use, one end of a cable 9 is secured to a transport bone segment 10, and the other end of the cable 9 is secured to the reel 8. The worm gear mechanism 4 rotates the reel 8 and winds up the cable 9, dragging the transport bone segment 10 toward a docking site.

As visible in Figure 4, the main body 2 has at least an opening 11 to expose a lateral portion of the reel 8. The cable 9 passes through the opening 11 and winds on the reel 8. A knob 12 is provided for driving the worm 5 into rotation.

As visible in Figure 5, the knob 12 is fitted on a same shaft 13 of the worm 5 and is therefore laterally placed on the same first end portion 7. The bolt 14 is at the second end portion 15.

The worm 5 comprises an externally threaded sleeve 16 which is locked on the shaft 13 by means of a set screw 17. The shaft 13 traverses a through-hole 18 with varying diameter of the main body 2. One end of the shaft 13, which emerges from outside the main body 2, is provided with an annular groove 19 for a locking O-ring 20.

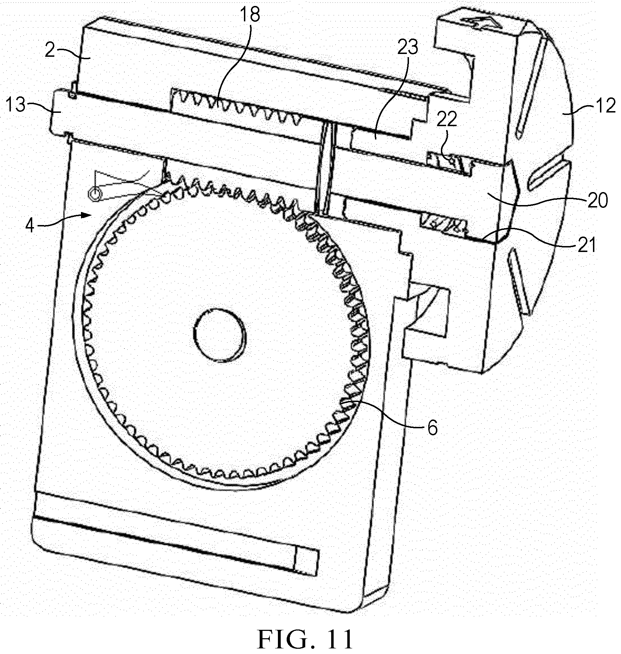

As clearly visible in Figure 11, the knob 12 is fixed at the other end of the shaft 13. The shaft 13 is in the form of a bolt, and a polygonal bolt head 20 of the shaft 13 mates with a corresponding seat 21 on the external face of the knob 12, such that the rotation of the knob 12 with respect to the shaft 13 is prevented. However, the knob 12 is free to move axially along the shaft 13. An elastic element 22 in the form of a spring is provided within the seat 21 between the knob 12 and the bolt head 20. Therefore, the knob 12 is pressed toward the main body 2 by the elastic element 22. The knob 12 is further provided with a nose portion 23 to be received in the through-hole 18 of the main body 2.

In the preferred embodiment herein described the outer outline of the knob 12 has a hexagon shape, and arrows are provided along an external rim of the knob 12 to indicate the direction along which the knob 12 is to be rotated. In this embodiment, the knob 12 is meant to be rotated by preset angular intervals of one sixth of a turn.

In the art of the present invention, bone segment transport is performed in steps where the distance of transport for each step is 0.25 to 0.50 mm performed two to four times a day. Mostly, a total of 1.0 mm of transport distance per day is desired. In this case, the cable pulling device 1 for bone transport may be configured to shorten the cable by 0.25 mm for each sixth turn of the knob 12. In the embodiment of the present invention, the worm 5 may be configured as a single-threaded worm, and the worm gear 6 may be provided with 60 teeth. Accordingly, the reduction ratio of the worm gear mechanism 4 is 60. The reduction ratio of the worm gear mechanism 4 and the diameter of the reel 8 can determine the amount of length change of the cable for a given activation increment of the knob according to the equation mentioned before. Therefore, the ideal diameter of the reel 8 can be determined to be 6*0.25*60/π, i.e., 28.648 mm. Moreover, this diameter of the reel 8 has been found to be large enough to allow the relatively stiff cable 9 to wind flat on it but not so large as to be bulky and require more than a single set of reduction gears.

The specific dimension mentioned above is only for illustrative purpose, it should be appreciated that other dimensions can be adopted by those skilled in the art without departing from the scope of the present invention.

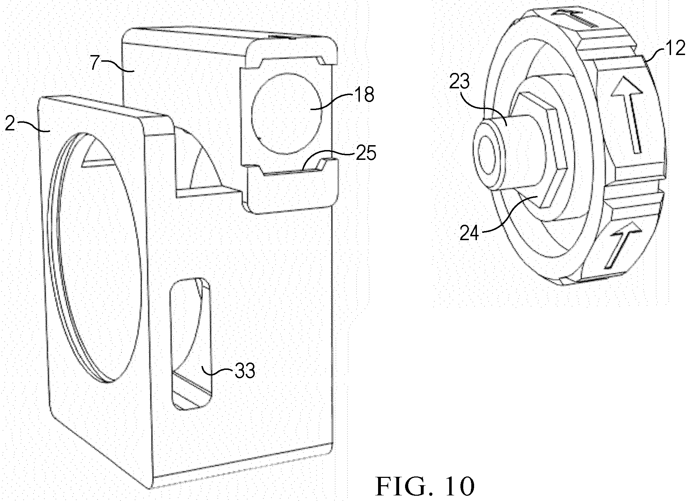

As visible in Figure 10, the knob 12 is provided with a regular polygon boss 24, and the main body 2 is provided with a recess 25 at a side of first end portion 7 thereof to receive the regular polygon boss 24 of the knob 12. The rotation of the worm 5 is prevented when the regular polygon boss 24 of the knob 12 is pressed into the recess 25 of the case 2. Since the elastic element 22 presses the knob 12 against the main body 2, the boss 24 of the knob 12 will be forced into the recess 25 of the case 2, forming a spring-loaded locking mechanism to prevent the rotation of the worm 5 and thus the worm gear 6 with the reel 8. Accordingly, it can be ensured that the knob 12 would be not accidentally turned between adjustments.

As seen above, the worm 5 can be rotated manually by rotating the knob 12. However, the present invention is not limited thereto. As an example, a motor (not shown) can be coupled to the shaft 13 of the worm 5 to drive the rotation of the worm 5. Further, a controller (not shown) can be also provided to control the motor. According to this non-illustrated embodiment, the controller can be configured to control an activation frequency of the motor and a rotation angle of the worm driven by the motor for each activation of the motor. Accordingly, the transport distance of the bone segment for each day can be controlled by the motor and the associated controller. In an embodiment of the present invention, the motor may be a stepper motor, and the controller may be a stepper motor driver, which are well-known by those skilled in the art.

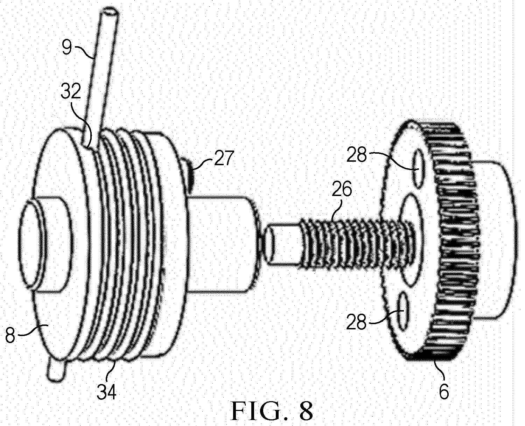

As visible for instance in Figure 8, the worm gear 6 is coaxially coupled with the reel 8. The axial screw 26 is used to fix the cable 9 into the reel 8. The reel 8 can be provided with a number of eccentric alignment pins 27 which engage with a corresponding number of alignment holes 28 of the worm gear 6. In the preferred embodiment, two holes and pins are provided: however, a different number of holes and pins can be used, and the hole and pins can be reversed on the reel and gear.

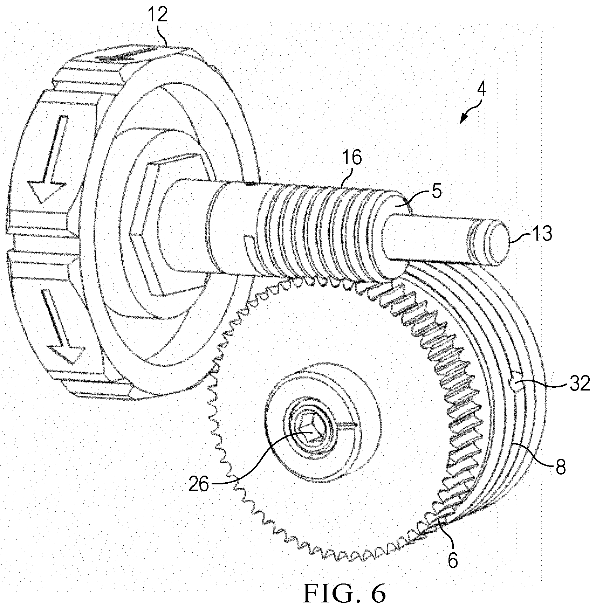

As visible in Figure 6, the worm gear 6 engages with the worm 5 to define the worm gear mechanism 4.

As visible in Figure 5, a circular opening 29, with varying diameter, is provided in the main body 2 to house the worm gear 6 and the reel 8, the latter being locked into position by a snap ring 30 placed over a washer 31.

As previously mentioned and visible in Figure 6, the reel 8 is provided with oblique radial hole 32 to facilitate the correct wire winding without overlapping. One end of the cable 9 is inserted into the radial hole 32, and then the cable 9 is clamped by the axial screw 26, thus securing it to the reel 8.

Any protruding part of the wire 9 can be cut at the opening 33 visible in Figure 10. With the rotation of the reel 8, the cable 9 is wind around the reel 8.

To ensure a uniform rate of transport of the transport bone segment 10, it is important to ensure that the cable 9 does not wind on top of itself. To this end, the reel 8 contains a spiral groove 34 around its outer surface to guide the cable 9 and prevent the cable 9 from overlapping and the oblique radial hole 32. A transport distance for each revolution of the reel 8 is a function of the circumference of the reel 8. In an embodiment where the reel 8 has a diameter of 28.648 mm, the circumference would be π*28.648 mm or 90 mm. In this case, each full turn of the reel 8 would shorten the cable 9 by 90 mm and move the transport bone segment 10 by the same amount.

In an embodiment of the present invention, which is not shown in the drawings, a force sensor may be attached to the cable 9 for sensing a tension force of the cable 9. The location of the fore sensor on the cable 9 can be determined according to the actual needs. According to the embodiment of the present invention, the force sensor can be used to sense the tension of the cable 9, which will allow to check if the formation of the callus takes place correctly. In addition, the amount of displacement can also be measured in order to support a biological function.

The worm gear 4 could have a ratchet mechanism which prevents reverse motion thereof. A ratchet mechanism of this kind, which is not strictly necessary in the preferred layout of the invention according to the enclosed Figures, could comprise a leaf spring acting on a locking pawl, which would be then biased towards the worm gear. Alternative forms of ratchet mechanisms could also be employed.

It should also be noted that any possible combination of the motor, the spring-loaded locking mechanism, and ratchet mechanism could be adopted in the present invention to prevent a reverse rotation of the worm gear 6 with the reel 8 under the load of the cable 9.

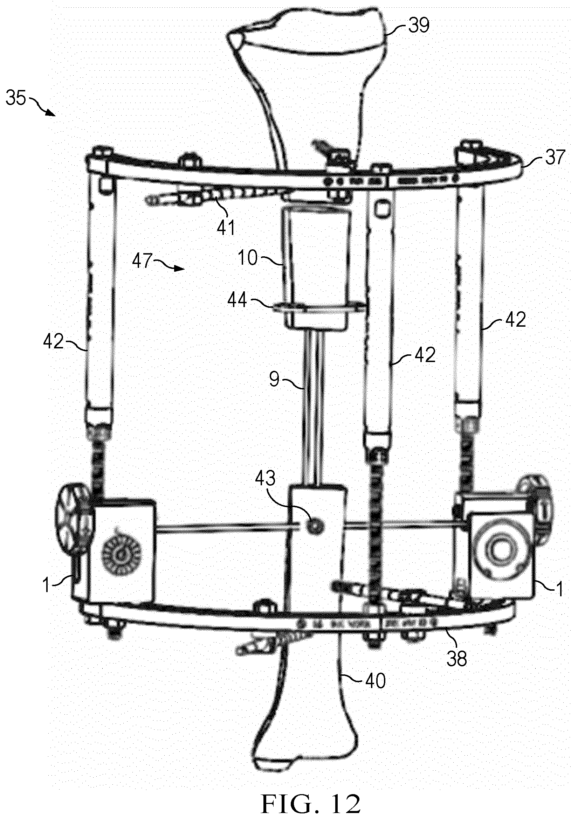

FIG. 12 shows an example of a bone transport system 35 which is provided with cable pulling devices 1 for bone transport and attached to bone segments according to an embodiment of the present invention.

The bone transport system 35 according to the invention is meant to be applied to a long bone of a patient; in the depicted embodiment the long bone is a tibia.

The bone transport system 35 comprises an external frame 47 which features a proximal ring 37 and a distal ring 38. The proximal ring 37 and the distal ring 38 are respectively anchored to a proximal fixed bone segment 39 and to a distal fixed bone segment 40 by means of tensioned wires and/or fixation pins 41. The proximal ring 37 is connected to the distal ring 38 by means of longitudinal rods 42. Preferably, longitudinal rods 42 are extensible.

A transport bone segment 10 is axially comprised between the proximal fixed bone segment 39 and the distal fixed bone segment 40.

The bone transport system 35 comprises two cable pulling devices 1 which are attached via their bolts 14 at diametrically opposite sites on the distal ring 38. The cable pulling devices 1 are therefore placed laterally and medially with respect to the distal fixed bone segment 40. A cable 9 is pulled between the two cable pulling devices 1 and follows a path which will be described in the following.

The bone transport system 35 further comprises means 43 for redirecting the cable 9 at the distal fixed bone segment. Preferably, said means 43 for redirecting the cable 9 take the form of a fulcrum pin or screw which is implanted in the distal fixed bone segment 40, following an anterior-posterior direction.

The cable 9 enters the transport bone segment 10 at its resected end and is attached thereto by means of an external loop 44, passing through at least one tangential hole made on the transport bone segment 10 into its intermedullary canal and bone defect area following the bone axis, enters the fixed distal bone segment 40 from the intermedullary canal and, after orthogonal redirection around the fulcrum pin or screw 43, exits that distal bone segment 40 medially and laterally.

As mentioned above, the cable 9 is symmetrically stabilized on the transport bone segment 10 by making the two ends exit axially into the medullary canal.

In the distal fixed bone segment 40, the two ends of the cable 9 rotate around the fulcrum pin 43 and exit the cortices one on one side and the other on the other side placed in the position of the devices 1 in line with the reels 8.

The bone transport system 35 is implanted via a surgical method which can be derived from the structural description given above.

According to the present invention, the improved cable pulling device for bone transport can provide sufficient stability of the transport bone segment, minimize number of external components between the proximal and distal external supports. The bone transport system according to the invention allows guiding independently the movement on the two ends of the cable, which allows to correct the possible angulation of the transport bone segment during transport relative to the proximal and distal fixed segments and simplifies transportation of the intercalary transport bone segment during the bone transport.

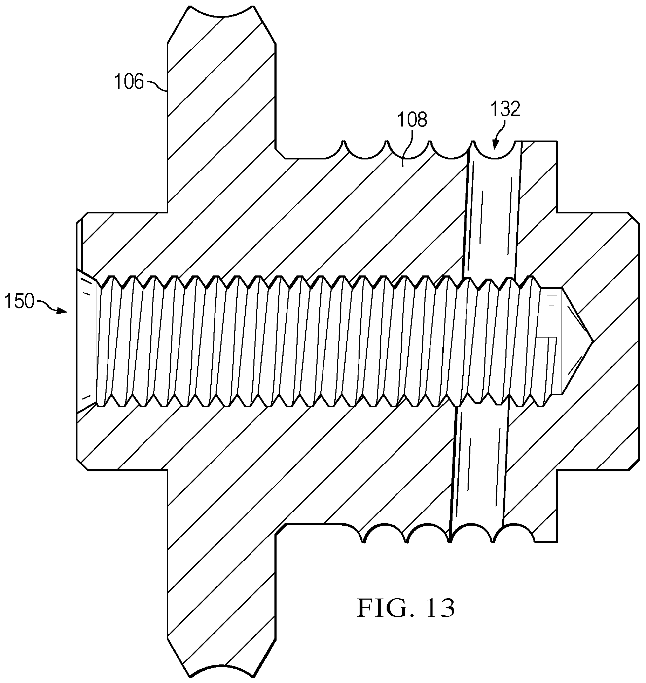

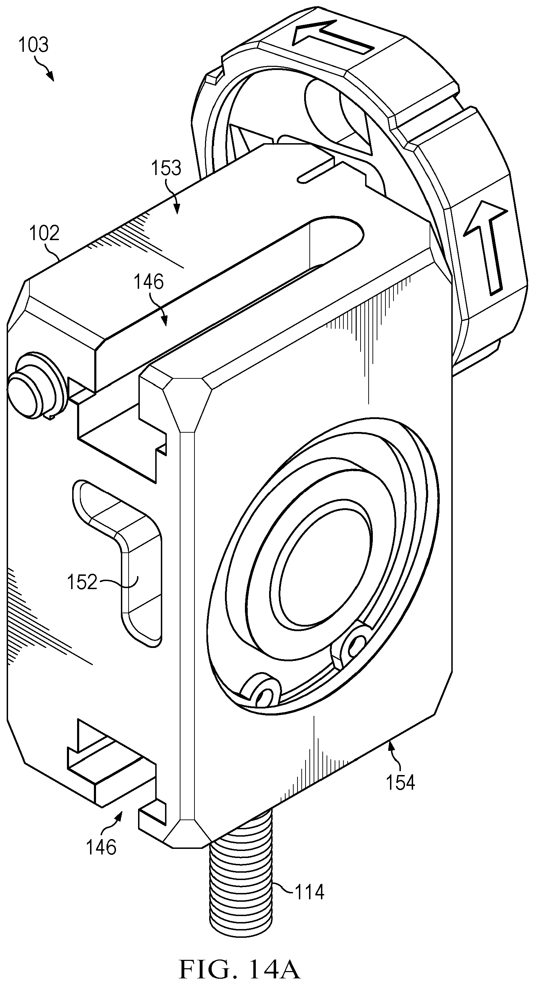

FIGS. 13-15 and 17-19 illustrate additional examples of cable pulling devices 103. Cable pulling devices 103 have some similar features and functions as cable pulling device 1 described above in relation to FIGS. 4-12. Accordingly, this description focuses on features of cable pulling devices 103 which may be different than those of cable pulling device 1 for the sake of brevity and to avoid redundant description. FIG. 13 shows a cross-sectional view of a reel and worm gear of a cable pulling device as shown in FIG. 14A. In the illustrated example, the worm gear 106 and reel 108 are formed as a single monolithic or integrated component, although they could be formed separately and coupled together. A blind bore 150 extends through the worm gear 106 and into the reel 108. A radial hole 132 extends through the reel 108 and intersects the blind bore 150. In some examples, the radial hole 132 may extend only to the blind bore 150 rather than passing through the entire diameter of the reel 108. In comparison to the reel 8 of FIG. 8, for example, the reel 108 may have a reduced outer diameter. In some examples, the diameter of the reel 108 may be approximately 10-20mm, for example, about 15mm. The reduced diameter of the reel 108 may occupy a smaller volume of space than the reel 8, providing room for additional features of the cable pulling device 103 without requiring the main body 102 to occupy additional space.

The main body 102 of the cable pulling device 103 shown in FIG. 14A includes a slot 146 at a first end 153 and a slot 146 at a second end 154. Each slot 146 may be configured to receive the head of a bolt and to prevent rotation of the bolt with respect to the main body 102. By providing a slot 146 at each of the first end 153 and second end 154, the cable pulling device may be mountable in two distinct orientations separated by 180°. Additionally, the cable pulling device may be simultaneously mounted to two external fixation rings. A first bolt (not shown) may extend from the slot 146 of the first end 153 to a first external fixation ring and a second bolt 114 may extend from the slot 146 of the second end 154 to a second external fixation ring. Providing double ring fixation of the main body 102 to an external fixation frame may provide improved mounting rigidity to the cable pulling device and resistance to bending of a bolt 114 of other components of the bone transport system.

The main body 102 includes a first window 152 formed in a side of the main body 102 opposite the knob 112. Typically, the cable pulling device 103 will be mounted to an external fixation system with the knob facing radially outward and the first window 152 facing radially inward toward to the bone being treated. The cable is typically passed through the first window 152 and secured to the reel 108 with a set screw (not shown) positioned in the blind bore 150. The first window 152 may have a vertical portion oriented between the first and second ends 153, 154. The vertical portion may be aligned with the radial hole 132 to facilitate positioning of the cable within the reel 108. The first window 152 may also include a horizontal portion extending along the width of the main body 102. As the cable is wound around the reel 108 within the helical groove of the reel, the cable may travel laterally along the direction of the horizontal portion of the first window 152. The vertical portion and horizontal portion of the first window 152 may form an L-shape.

The slots 146 are open on an end facing the wall in which the first window 152 is disposed. As will be appreciated, the position of the first window 152 and the cable passing therethrough results in a force biasing the cable pulling device 103 toward the bone of the patient when the cable is tensioned. Having the slots 146 open in the direction of the biasing force may help prevent the main body 102 from sliding off of the head of the bolt(s) 114. In some examples, rather than slots 146 being sized and shaped to receive and retain the head of a bolt, one or more slots 146 may be sized and shaped to receive and retain a nut configured to receive a bolt extending through an aperture of an external fixation ring. In some examples, a hexagonal recess may be formed in the first end 153 or second end 154 to receive and retain a nut. In other examples, a threaded bore configured to receive a bolt may be formed integrally into the main body 102.

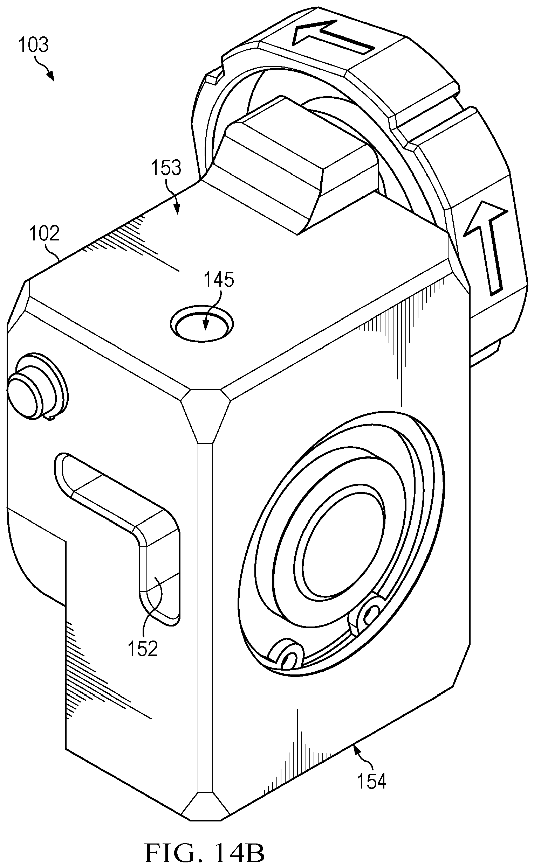

FIG. 14B shows another example of a cable pulling device 103. As compared to the example of FIG. 14A, the cable pulling device 103 of FIG. 14B does not include slots 146. Rather the main body of the cable pulling device 103 of FIG. 14B includes one or more bores 145 formed into each of the first end and second end of the main body. These bores may be threaded to engage a bolt secured to an external fixation ring. Alternatively, these bores may be configured to receive fasteners of a mounting bracket (not shown). Such a mounting bracket may include a slot 146 and may be removably attachable to the first end and/or the second end.

Referring to FIG. 14B, the main body of the example cable pulling device 103 includes a curved external wall. Similarly, the main body includes a curved internal wall conforming to the shape of the reel 108. In this regard, the cavity formed in the main body housing the reel 108 may be cylindrically shaped and have a radium exceeding the radius of the reel 108 by an amount that is equal to or greater than the thickness of the cable but less than twice the radius of the cable. In this regard, the cable may be prevented from layering on top of itself as it is wound around the reel 108 as insufficient space to stack two layers of cable. In some embodiments, two layers of cable may be permitted and the cavity may be sized to permit two layers but prevent three layers.

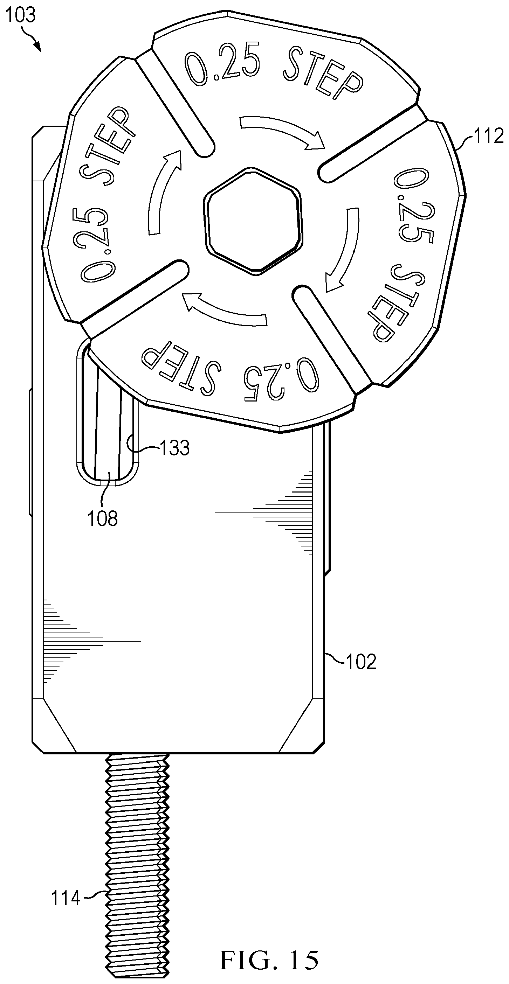

FIG. 15 shows a front view of the cable pulling device of FIG. 14A. A second window 133 is formed in a wall of the main body 102 disposed opposite the first window 152 (see FIG. 14A). When the cable is initially fed through the first window 152 and the radial hole 132 in the reel 108, an end of the cable may extend out the second window 133. Prior to or after securing the cable to the reel 108 with a set screw, a user may cut the cable to trim off an excess length protruding through the second window 133. This may prevent any excess cable length which would simplify the winding and reduce the likelihood of an undesirable layering of the cable as it is wound around the reel 108.

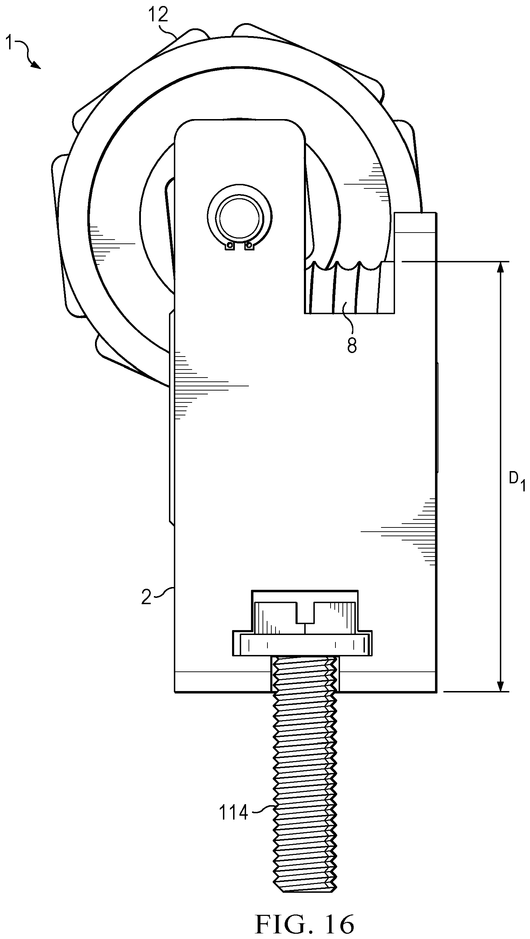

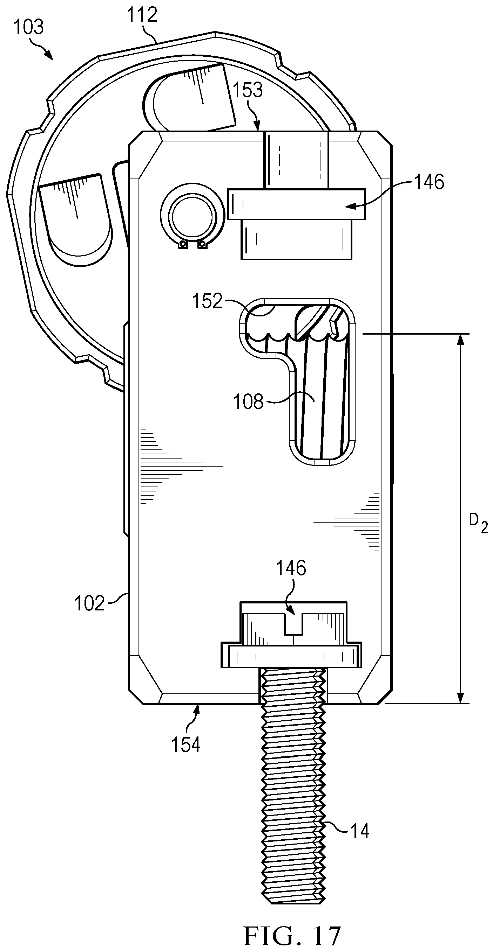

FIG. 16 shows a rear view of the cable pulling device 1 of FIG. 4 and FIGS. 17-18 show a rear view of the cable pulling device 103 of FIG. 14A. As will be appreciated by comparing FIG. 16 with FIGS. 17-18, providing a reduced diameter of the reel 108 as compared to the reel 8 may provide space in the main body 102 to accommodate a slot 146 at the first end 153 without increasing the overall height of the main body 102. Furthermore, reducing the diameter of the reel 108 as compared to reel 8 may reduce the distance D₂ between the second end 154, which may be connected to an external fixation ring, and the top of the reel 108 as compared to a similar dimension D₁ of the cable pulling device 1. Because the cable will extend horizontally from the top of the reel to the bone of patient and the cable is under tension, this distance D₁ or D₂ is effectively a moment arm such that positioning the top of the reel closer to the external fixation ring may reduce stress on the bolt 114. In some implementations, the reel and body may be arranged to permit the cable to wind from below the reel.

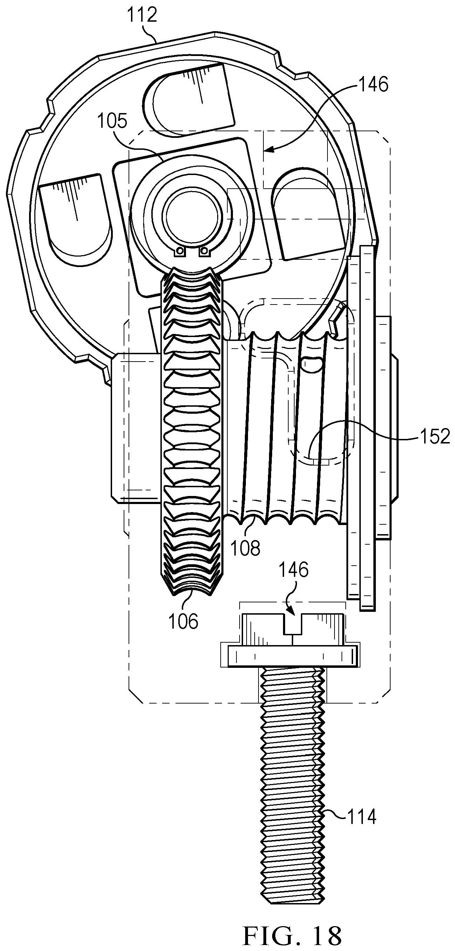

A cable pulling device, such as cable pulling device 103 shown in FIG. 18, may include any suitable driver. In the illustrated embodiment, the driver includes a worm screw 105 manually rotatable by knob 112 and also includes the worm gear 106 coupled to reel 108. In some embodiments, a driver may include a motor configured to directly or indirectly drive the reel 108. For example, a motor may be connected to the worm screw 105 which, in turn, rotates the worm gear 106 and reel 108. In another example, the worm gear 106 may be omitted and a motor may directly drive the reel 108 with the drive shaft of the motor being positioned along the axis of rotation of the reel. A driver of the current disclosure may include any suitable structure(s) to facilitate manual or motorized application of force or torque to rotate the reel 108, including but not limited to one or more of a transmission, a clutch, a motor, a series of gears, a worm drive mechanism, cables and pulleys, etc.

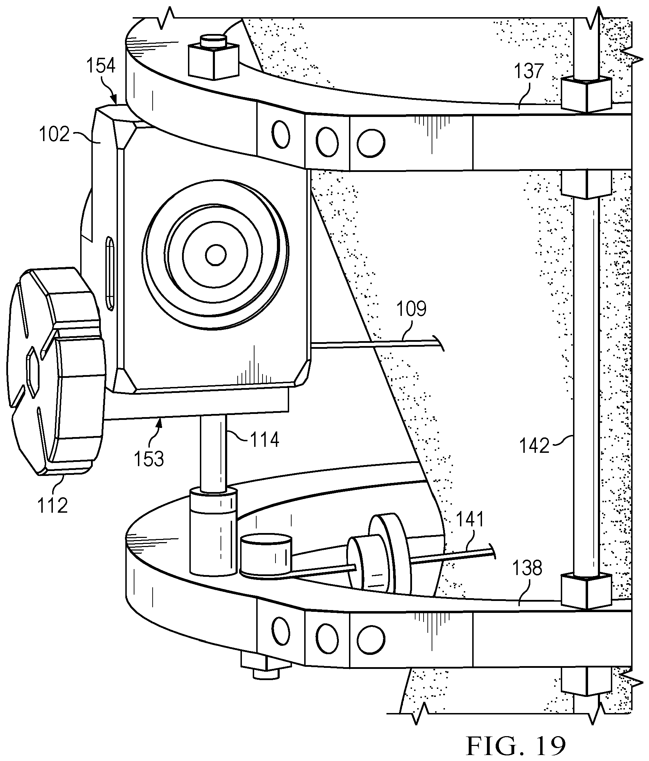

FIG. 19 shows a perspective view of a bone transport system including a cable pulling device 103. As illustrated, a first bolt 114 extends from the first end 153 of the main body 102 and is secured to the distal ring 138 which is secured to a distal fixed bone segment. A second bolt extends from the second end 154 of the main body and is secured to the proximal ring 137 which is secured to a proximal fixed bone segment. The cable 109 extends horizontally from the main body 102 to the distal fixed bone segment. Tension in the cable 109 tends to pull the main body 102 toward the distal fixed bone segment. Utilizing two bolts, one extending proximally and one extending distally, to secure the main body 102 to two external fixation rings 137 and 138, instead of one fixation ring, may reduce the moment forces and aid in distributing the loading from the cable 109 and prevent deformation in the cable bone transport system. While in the illustrated example, the second end 154 of the main body 102 is in contact with the proximal ring 137 and the first end 153 is spaced from the distal ring 138, in some examples the first end 153 may be in contact with the distal ring 138 and the second end 154 may be spaced from the proximal ring 137. Further, both ends 153, 154 of the main body 102 may be in contact with a respective ring 137, 138 or both ends 153, 154 may be spaced from a respective ring 137, 138. Additionally, while only one cable pulling device 103 is shown in FIG. 19, it will be appreciated that a second cable pulling device 103 may be similarly situated on an opposing side of the rings.

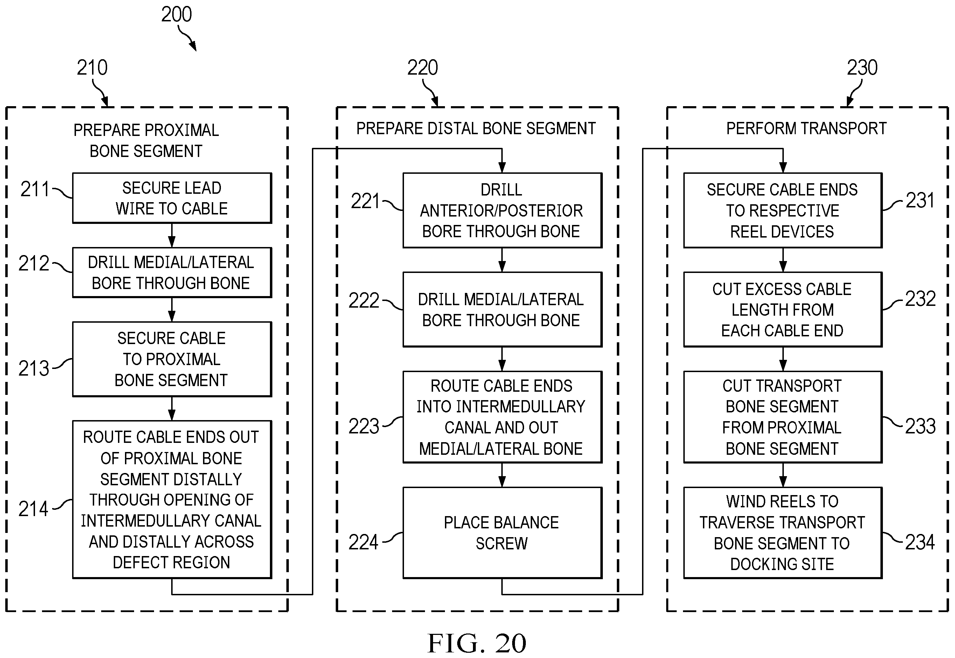

FIG. 20 is a flowchart of an example method of installing a bone transport system. The method 200 includes three phases including preparing the proximal bone segment 210, preparing the distal bone segment 220, and preparing the cable pulling devices and performing the bone transport 230. It should be appreciated that the processes of each phase need not be performed in the example sequence illustrated. For example, some processes for preparing the distal bone segment may be performed before or simultaneously with some processes for preparing the proximal bone segment. Similarly, each process within a given phase need not be performed in the illustrated order.

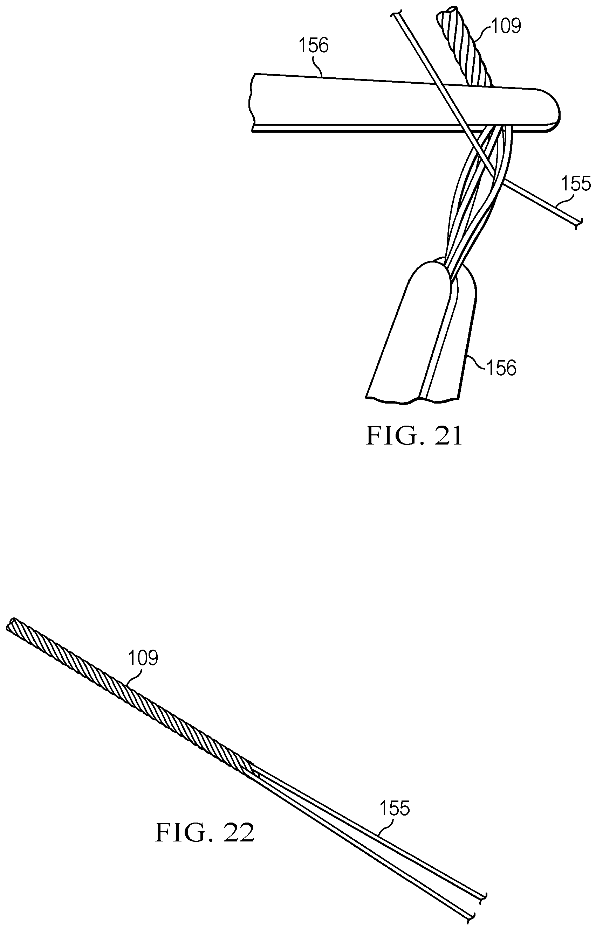

At process 211, a lead wire may be secured to a cable. A lead wire may comprise any suitable string, suture, metal wire, thread, leader, etc. that is more flexible than the cable itself. In this regard, the lead wire may be easier to route through a tortuous path and can then be pulled to lead the cable through the path. In some examples, securing the lead wire to the cable may include untwisting the strands of the cable near an end of the cable as shown in FIG. 21. Pliers 156 may be used to grip the cable 109 to isolate untwisting of the strands to the region between the pairs of pliers 56. When a gap is opened between the strands, the lead wire 155 may be passed through the gap to secure the lead wire 155 to the cable 109. In some examples, the pliers 156 may be used to retwist the strands of the cable and pinch the lead wire 155 between adjacent strands. FIG. 22 illustrates a cable 109 with an attached lead wire 155. In some examples, the lead wire 155 may be further secured to the cable 109 with an adhesive such as glue or tape. In some examples, a curable resin may be applied where the lead wire 155 passes through the cable 109 and a UV light or heat source may be used to cure the resin and affix the lead wire 155 to the cable 109. In some examples, a sleeve may be slid over the lead wire 155 and onto the cable 109. The sleeve may then be crimped in place to affix the lead wire 155 to the cable 109. Various combinations of these techniques may be used to ensure the lead wire 155 remains secured to the cable 109.

At process 212, a first bore is drilled through the proximal bone segment. In some examples, the first bore may extend in a medial-lateral direction although any suitable orientation may be used. The portion of the proximal bone segment through which the first bore is drilled may be the transport bone segment. However, at this stage, the transport bone segment may still be fixed to the proximal bone segment.

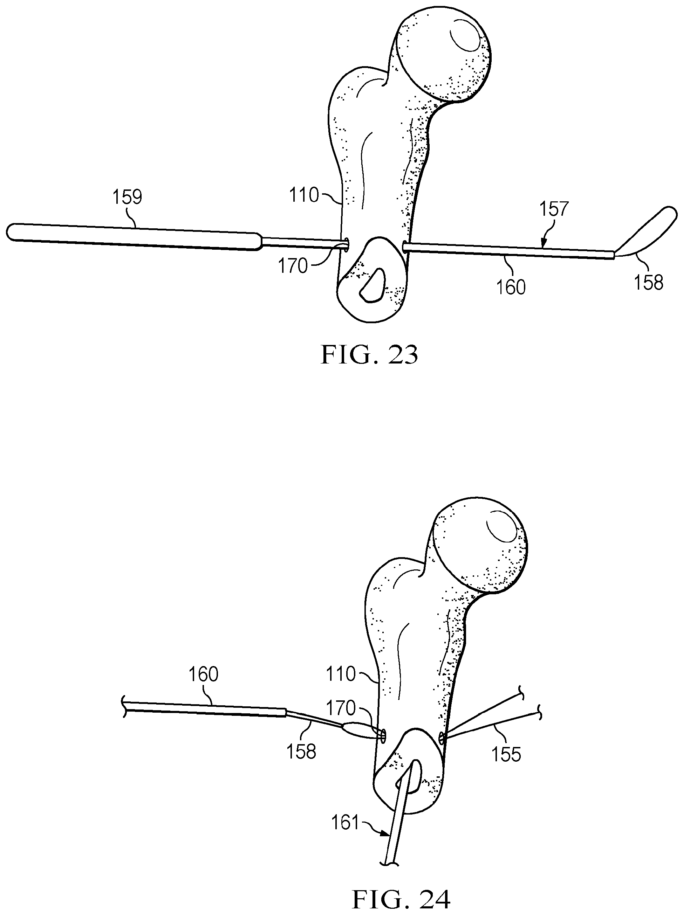

At process 213, a cable is secured to the proximal bone segment. As shown in FIG. 23, a suture puller or snare 157 may be used to facilitate routing of the cable. The snare 157 includes a loop 158 formed of a thread, wire, or other thin flexible material, a handle 159, and a shaft 160 extending between the loop 158 and handle 159. The loop 158 and shaft 160 may be passed through both cortices of the transport bone segment 110 in the first bore 170. The lead wire 155, when used, is the passed through the loop 158 and the snare 157 is retracted through the first bore 170, as shown in FIG. 24, pulling the lead wire 155 through the first bore 170. If a lead wire is not used, the snare 157 may directly pull the cable 109. With the lead wire 155 spanning the intermedullary canal of the proximal bone segment 110, a hook 161 may be inserted axially into the intermedullary canal to engage the lead wire 155. The hook 161 may then be pulled from the intermedullary canal, pulling the lead wire 155 with it.

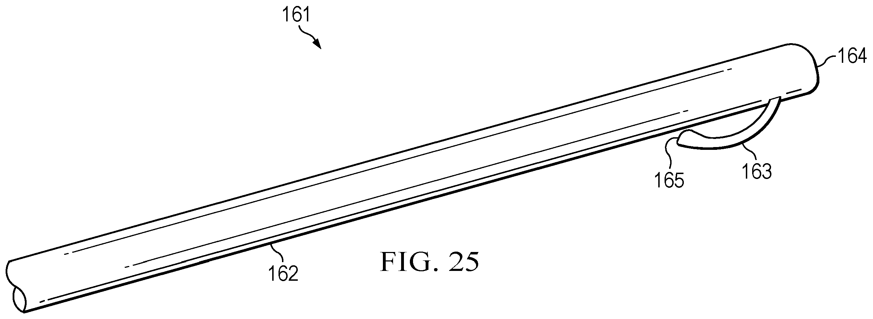

FIG. 25 illustrates an example of a medical tool which may be used as the hook 161. A shaft 162 extends to a distal tip 164. The distal tip 164 may be atraumatic or blunt to prevent undesirable damage to tissue in the intermedullary canal. A clip 163 may be positioned adjacent to the distal tip 164 and extend from a point of attachment to the shaft 162 away from the distal tip 164 in a proximal direction. The clip 163 may be curved and may be flexible. In some examples, a proximal end of the clip 163 may be spaced apart from the shaft 162 to allow the lead wire 155, when slid along the shaft 162 as the hook 161 is retracted, to pass between the clip 163 and the shaft 162. In the illustrated example, the proximal end of the clip 163 has an angled surface 165 configured for contact with the lead wire 155 such that the lead wire 155 may lift the clip 163 away from the shaft 162 and become trapped within the passage between the shaft 162 and clip 163. A user may maintain tension in the lead wire 155 during retrieval with the hook 161 to facilitate capture of the lead wire 155 within the clip 163. In some examples, the shaft 162 may be flexible to aid in positioning the clip 163 into the intermedullary canal oriented proximal-distal with access from the medial or lateral side. In some examples, the shaft 162 comprises a first diameter along a majority of the shaft but a second diameter, smaller than the first diameter, along at least a portion of the shaft on which the clip 163 is disposed. The reduced diameter at the location of the clip 163 may allow the overall diameter of the hook 161 to remain relatively narrow despite the additional width of the clip 163.

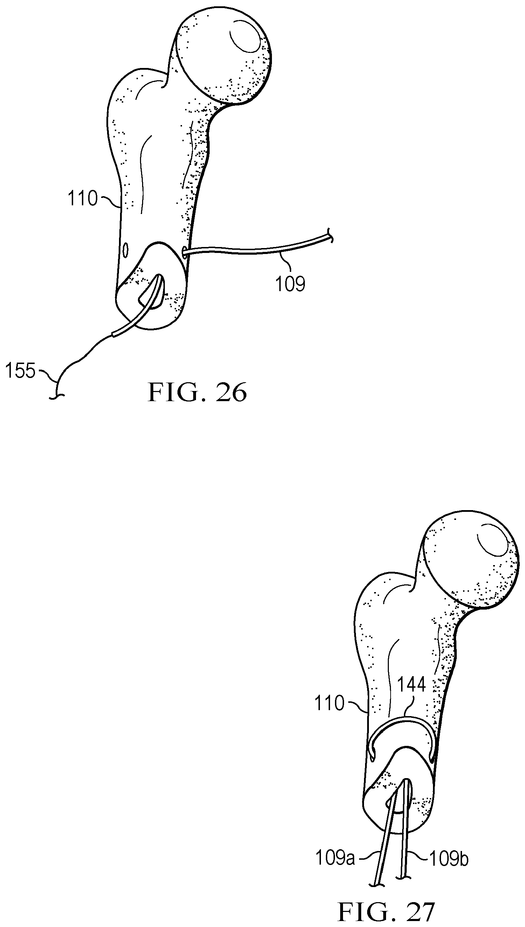

At process 214, the lead wire 155 may then be pulled distally to feed a first end of the cable out of the proximal bone segment through an opening of the intermedullary canal and distally into the bone defect region as illustrated in FIG. 26. The process may then be repeated to feed a second end of the cable through the intermedullary canal and into the bone defect region, forming an external loop 144 in the cable 109 with a first segment 109a and second segment 109b each extending distally from the intermedullary canal of the transport bone segment 110 as shown in FIG. 27. At this stage, preparation of the proximal bone segment 210 may be complete.

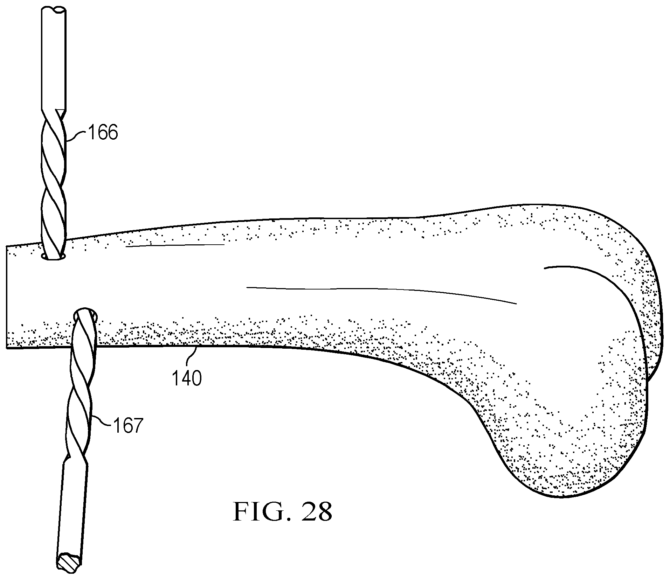

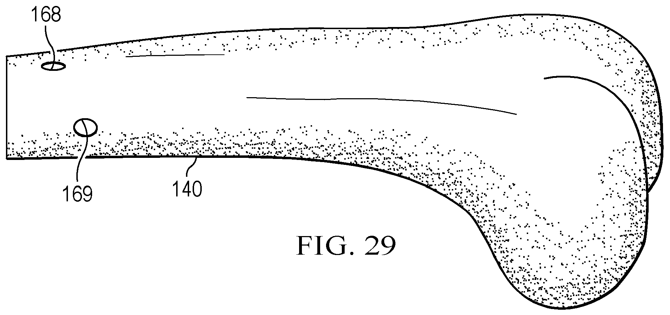

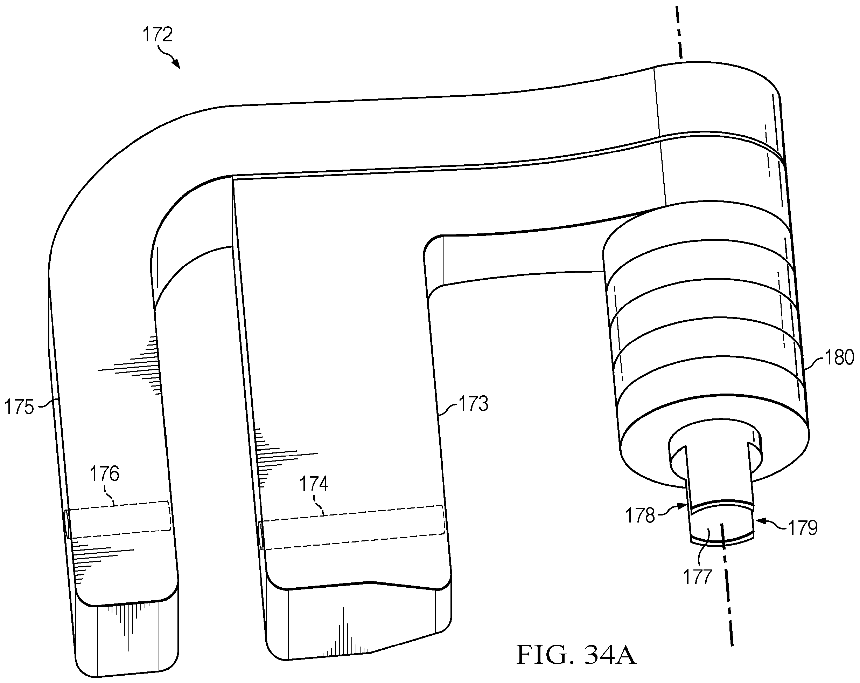

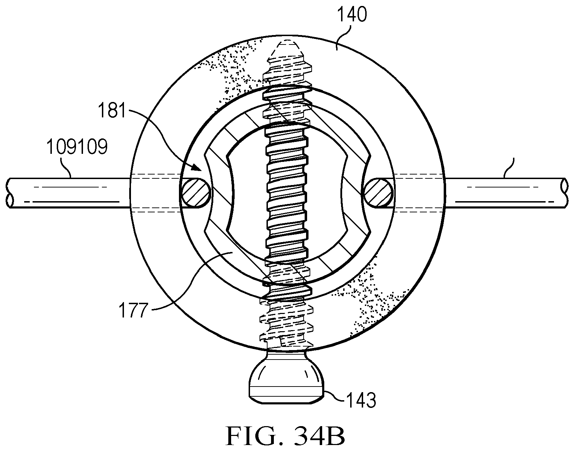

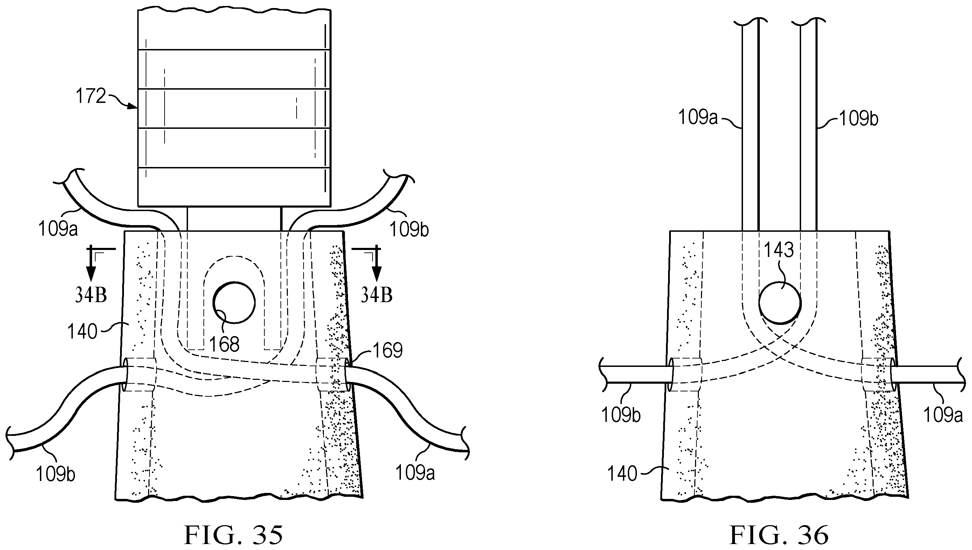

At process 221, a second bore is drilled through the distal bone segment 140 and at process 222 a third bore is drilled through the distal bone segment 140, as shown in FIGS. 28-29. It should be appreciated that processes 221 and 222 may be performed in any order. A first drill bit 166 may be used to drill the second bore 168 and a second drill bit 167 may be used to drill the third bore 169. The second bore 168 is preferably oriented orthogonally to the third bore 169, typically in an anterior-posterior direction. In this regard, the third bore 169 may be oriented parallel to the first bore 170 in the transport bone segment 110. The second bore 168 and third bore 169 may overlap or be completely offset from one another. Typically, it is preferable to position each bore 168 and 170 at least 10mm from the cut plane of the respective bone segment to ensure sufficient strength during tensioning of the cable 109. In some example implementations, a drill guide or jig may be used to drill one or more of the bores as is described further below in relation to FIG. 34A, 34B, and35.

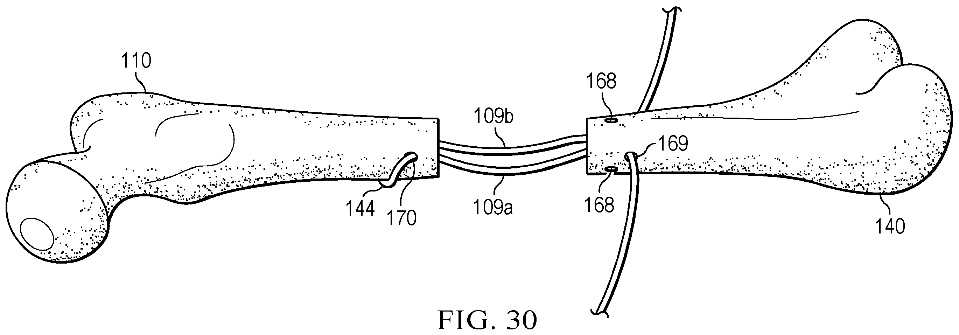

At process 223, the ends of the cable 109 are routed into the intermedullary canal of the distal bone segment 140 and out through respective ends of the second bore 169, as shown in FIG. 30. The snare 157 and/or hook 161 may again be used to aid in this process. As soft tissue and fluids may obstruct the surgeon's view, it may be difficult to discern the first cable segment 109a from the second cable segment 109b. In order to properly facilitate controlled transport of the transport bone segment 110, it may be important route the two cable segments longitudinally between the transport bone segment 110 and distal bone segment 140 without crossing one another. In this regard, it is contemplated that each segment 109a, 109b may have a property that differs from the other to facilitate visually distinguishing each from the other. For example, the first cable segment 109a may be painted a first color and the second cable segment 109b may be unpainted or painted a second color. Alternatively, a sleeve may be positioned over one of the segments while the other remains bare or sleeves with different colors or different markings may be positioned over each respective segment 109a, 109b. Alternatively, different surface finishes may be used such that the first cable segment 109a is glossy or smooth while the second cable segment 109b is rough or dull. In some examples, a lead wire secured to one end of the cable may have a first color and a lead wire secured to the other end of the cable may have a second color. The visual distinction between the first cable segment 109a and second cable segment 109b may aid the surgeon in orienting the first cable segment 109a from the medial end of the first bore 170 to enter the intermedullary canal of the distal bone segment 140 on the medial side and in orienting the second cable segment 109b from the lateral end of the first bore 170 to enter the intermedullary canal of the distal bone segment 140 on the lateral side.

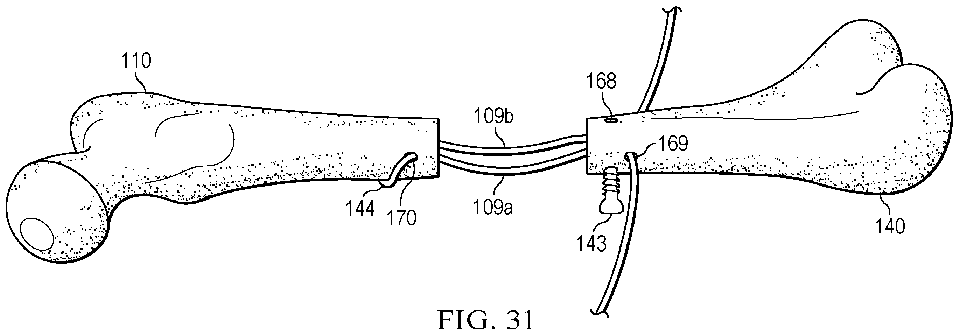

At process 224, a balance screw 143 may be placed into the second bore 168, as shown in FIG. 31 in a manner spanning the intramedullary canal. The balance screw 143 may act as a fulcrum pin to translate horizontal force on the cable 109 into vertical force to pull the transport bone segment 110 during transport. In this regard the first cable segment 109a may pass distally of the balance screw 143 and exit the distal bone segment 140 through the lateral end of the third bore 169 and the second cable segment 109b may pass distally of the balance screw 143 and exit the distal bone segment 140 through the medial end of the third bore 169, as shown in FIG. 36.

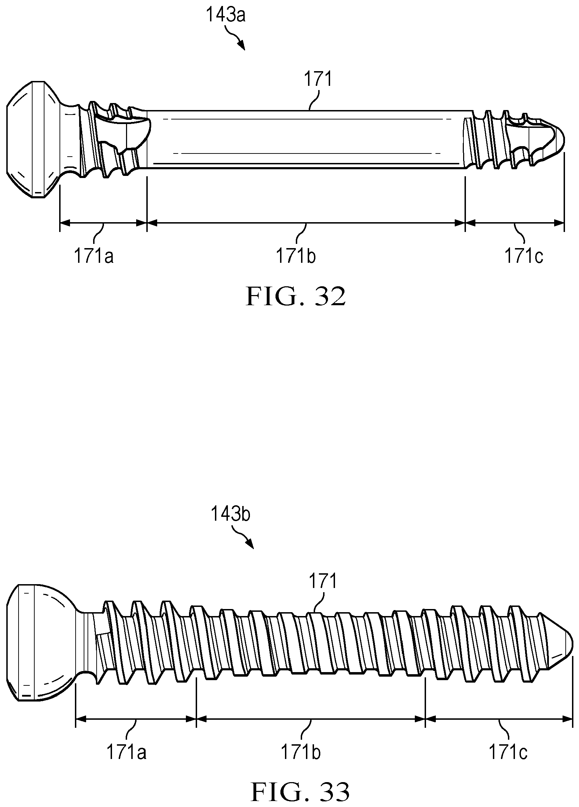

An example of a balance screw 143a is shown in FIG. 32. The balance screw 143a includes a shaft 171 with a first threaded region 171a, a second threaded region 171c, and an intermediate region 171b positioned between the first and second threaded regions. The intermediate region 171b may be smooth to reduce friction between the cable 109 and shaft 171. In some examples, the intermediate region 171b may include cable guides to prevent the first cable segment 109a and second cable segment 109b from binding together. FIG. 33 illustrates an example of a balance screw 143b having threads in the intermediate region 171b which have a smaller diameter and which are dulled as compared to the threads in the first and second threaded regions 171a, 171c. The helical grooves between the threads in the intermediate region 171b may act as cable guides to retain the first cable segment 109a in a first helical groove and the second cable segment 109b in a second helical groove. In some examples, the intermediate region 171b may be primarily smooth such as in FIG. 32 but may have first and second cable guides in the form of transverse grooves forming a recessed ring around the shaft 171. In some examples, the intermediate region 171b may have a first sleeve or roller and a second sleeve or roller to facilitate movement of the first and second cable segments 109a, 109b in opposing directions while reducing friction between the cable 109 and balance screw 143. A drill guide or jig, such as that used to drill the second bore 168 and third bore 169, may be used to prevent the balance screw 143 from damaging the cable 109 during insertion as well as ensuring the first and second cable segments 109a, 109b are properly positioned to cross distally of the balance screw 143, as is discussed further below in relation to FIGS. 34B and 35. At this stage, preparation of the distal bone segment 220 may be complete.

At process 231, the ends of the cable 109 may be secured to the reels of respective cable pulling devices (e.g., cable pulling device 103). A first cable pulling device may be secured to a first external fixation ring and a second cable pulling device may be secured to the first external fixation ring at a position diametrically opposed to the first cable pulling device. After passing the ends of the cables through the radial hole in each reel, the cable ends may be pulled apart in a radially outward direction and secured the cable with respective set screws.

At process 232, the excess length of cable protruding from each reel may then be cut off. The strands of the cable 109 may be secured to prevent fraying. For example, a strip of tape may be wrapped around the cable or a sleeve may be crimped onto the cable, and the cut may be made through the tape or crimped sleeve to ensure the ends of the strands are bound. In some examples, a rapid-setting adhesive such as a cyanoacrylate may be used to bind the ends of the strands.

At process 233, in order to permit transport of the transport bone segment 110, the transport bone segment 110 is cut from the proximal fixed bone segment. It will be appreciated that process 233 may be performed at any suitable stage earlier in the method 200 but must be performed before process 234.

At process 234, the reels of the cable pulling devices are wound. In the illustrated examples, the knob of the driver may be manually rotated to drive the worm screw and, in turn, the worm gear. In other examples, a controller may be used to initiate rotation of a motorized driver. Winding the reels of the cable pulling devices tensions the cable between the transport bone segment and the fixed distal bone segment to urge the transport bone segment toward the fixed bone segment.

FIG. 34A shows a drill guide that may be used in drilling one or more of the first, second, and third bores 168-170. The drill guide 172 includes a shaft 177. One or more spacer washers 180 may be positioned around the shaft 177 to set a desired length of shaft 177 that is exposed. The exposed length of shaft 177 is configured to be inserted axially into the intermedullary canal of a bone. The spacer washer 180 nearest the exposed tip of the shaft 177 may act as a guide surface to limit insertion of the shaft 177 into the intermedullary canal to the exposed length which corresponds to a desired depth in the end of the bone. That is, the spacer washers 180 may have a diameter that exceeds that of the intermedullary canal to prevent the spacer washers from entering the canal. In this regard, a guide surface formed by the spacer washer 180 nearest the tip of the shaft 177 abuts the cut plane of the bone to control the insertion depth of the shaft 177. In some examples, the spacer washers 180 may be omitted and the guide surface may be integrated into the drill guide 172 at a fixed distance from the tip of the shaft rather than an adjustable amount. A distal end portion of the shaft 177 includes slots 178, 179 positioned on opposing sides of the shaft 177. In the illustrated example, the slots 178, 179 have an open end at the tip of the shaft 177 but in some examples may be enclosed. Alternatively, two circular apertures spaced along the longitudinal axis of the shaft may be formed in place of slots 178, 179.

The drill guide 172 includes a first handle 173 and a second handle 175, each including a grip portion oriented parallel to the shaft 177 and a bridge connecting the grip portion to the shaft. A first drill bore 174 sized to receive a drill bit or sleeve through which a drill bit may be extended is positioned in the first handle 173. A similar second drill bore 176 is positioned in the second handle 175. In the illustrated configuration, both the first drill bore 174 and the second drill bore 176 are axially aligned with the slots 178, 179 in the shaft 177. The first drill bore 174 and second drill bore 176 may be axially offset along the longitudinal axis of the shaft 177 or may be radially aligned. Either the first handle 173 or the second handle 175 may be configured to swivel about a longitudinal axis of the shaft 177 and the other of the first handle 173 or second handle 175 may be fixed with respect to the shaft 177. The drill guide 172 may include a stopper configured to limit rotation of the handles with respect to one another to a maximum of 90° about the longitudinal axis of the shaft 177. In this regard, the first drill bore 174 may be used to drill the second bore 169 in the distal bone segment 140 in a first orientation and the second handle 175 may be positioned to align the second drill bore 176 to drill the third bore 168 in the distal bone segment in an orthogonal orientation. It should be appreciated that the first bore 174 may be positioned in the second handle 175 and the second bore 176 may be positioned in the first handle 173. In this regard, the first handle 173 may be shorter than second handle 175 providing clearance for a drill bit to extend through the second handle 175 and into the slots 178 and 179 without passing through the first handle 173. In some examples, the end of the shaft may include two additional slots oriented 90° with respect to the slots 178, 179 to allow passage of a drill bit orthogonal to the slots 178, 179.

As shown in the cross-section of FIG. 34B, the shaft 177 may include diametrically opposed grooves 181 along a portion of its length. The grooves 181 may be sized to receive the cable 109. Prior to inserting the balance screw 143, the drill guide 172 may be positioned in the distal bone segment with the first and second cable segments in a respective one of the grooves 181. In this regard, the shaft 177 may push the cable segments toward the cortices of the bone to prevent the balance screw 143 from piercing or damaging the cable 109, as shown in FIG. 35. Additionally, positioning the cable segments in the grooves 181 during insertion of the balance screw 143 may help ensure the cable segments are positioned on their respective sides of the intermedullary canal to properly cross distally of the balance screw 143 as shown in FIG. 36.

Although specific examples have been illustrated and described above, those of ordinary skill in the art will appreciate that an arrangement to achieve the same results can be substituted for the specific embodiments shown. This disclosure is intended to cover adaptations or variations of one or more embodiments of the present disclosure. It is to be understood that the above description has been made in an illustrative fashion, and not a restrictive one. The scope of one or more examples of the present disclosure should be determined with reference to the appended claims, along with the full range of equivalents to which such claims are entitled.

## Claims

1. A bone transport system (35), comprising:
an external fixation frame (47) configured to be attached to at least one fixed bone segment (40; 140); and
an element for redirecting a cable (9; 109) configured to be attached to the fixed bone segment (40; 140) and to redirect the cable (9; 109) departing from at least a cable pulling device (1; 103) towards a transport bone segment (10; 110);
**characterized in that** it further comprises the at least one cable pulling device (1; 103) solidly attached to the external fixation frame (47) comprising:
a main body (2; 102) secured to the external fixation frame (47) solidly attached to the at least fixed bone segment (40; 140);
a reel (8; 108) rotatable about said main body (2; 102), for winding up the cable (9; 109) to be secured to the transport bone segment (10; 110), thus moving said transport bone segment (10; 110) towards said fixed bone segment (40; 140);
a transmission mechanism driving the reel (8, 108) in order to wind up the cable (9; 109).

2. The bone transport system (35) according to claim 1, wherein the transmission mechanism is a worm gear mechanism (4).

3. The bone transport system (35) according to claim 2, wherein the transmission mechanism comprises at least one irreversible kinematic pair.

4. The bone transport system (35) according to claim 1, wherein the worm gear mechanism (4) comprises:
a worm (5; 105) rotatably fixed to the main body (2; 102);
and a worm gear (6; 106) rotatably fixed to the main body (2; 102) and engaging with said worm (5; 105);
said reel (8; 108) rotating integrally with said worm gear (6; 106).

5. The bone transport system (35) according to claim 4, wherein it further comprises a knob (12; 112) to transmit a rotating motion to said worm (5; 105).

6. The bone transport system (35) according to claim 5, further wherein said knob (12; 112) has a locking mechanism that locks the rotation of the knob (12; 112) with respect to the main body (2; 102) at given locking angular positions of the knob (12; 112).

7. The bone transport system (35) according to claim 6, wherein the locking angular positions corresponds to fixed angular increments in the rotation of the knob (12; 112).

8. The bone transport system (35) according to claim 7, wherein the locking mechanism comprises a regular polygon boss (24) and a mating recess (25) respectively provided on the knob (12) and on the main body (2), or the other way around, wherein the rotation of the worm (5) is prevented when the regular polygon boss (24) is pressed into the recess (25), the locking mechanism further comprising an elastic element (22) that biases the knob (12) towards the main body (2).

9. The bone transport system (35) according to claim 4, further comprising a motor coupled with the worm (5; 105) to drive the rotation of the worm (5; 105) and a controller for controlling the motor, wherein the controller is configured to control an activation frequency of the motor and a rotation angle of the worm (5; 105) driven by the motor for each activation of the motor.

10. The bone transport system (35) according to claim 1, wherein the reel (8; 108) comprises a spiral groove (36) on its external surfaces to receive the cable (9; 109) as it winds up, preventing overlapping.

11. The bone transport system (35) according to claim 1, further comprising a force sensor directly or indirectly coupled to the cable (9; 109) for sensing a tension force acting on the cable (9; 109) .

12. The bone transport system (35) according to claim 1, wherein said element for redirecting the cable (9; 109) is a fulcrum pin or screw (43; 143a; 143b).

13. The bone transport system (35) according to claim 12, comprising two cable pulling devices (1; 103) solidly attached to the external fixation frame (47) in diametrically opposite positions, wherein the two cable pulling devices (1; 103) hold the opposite ends of a same cable (9; 109) .

## Patentansprüche

1. Knochentransportsystem (35), mit
einem Fixateur externe (47), der so konfiguriert ist, dass er an mindestens einem festen Knochensegment (40; 140) befestigt werden kann; und
einem Element zum Umlenken eines Kabels (9; 109), das so konfiguriert ist, dass es an dem festen Knochensegment (40; 140) befestigt werden kann und das Kabel (9; 109), das von mindestens einer Kabelziehvorrichtung (1; 103) aus in Richtung eines Transportknochensegments (10; 110) läuft, umlenkt;
**dadurch gekennzeichnet, dass** es ferner die
mindestens eine Kabelziehvorrichtung (1; 103) aufweist, die fest mit dem Fixateur externe (47) verbunden ist und Folgendes aufweist:
einen Hauptkörper (2; 102), der an dem Fixateur externe (47) befestigt ist, der fest mindestens an dem festen Knochensegment (40; 140) angebracht ist;
eine Spule (8; 108), die um den Hauptkörper (2; 102) drehbar ist, um das an dem Transportknochensegment (10; 110) zu befestigende Kabel (9; 109) aufzuwickeln, wodurch das Transportknochensegment (10: 110) in Richtung des festen Knochensegments (40; 140) bewegt wird;
einen Getriebemechanismus, der die Spule (8; 108) antreibt, um das Kabel (9; 109) aufzuwickeln.

2. Knochentransportsystem (35) nach Anspruch 1, bei dem der Getriebemechanismus ein Schneckengetriebemechanismus (4) ist.

3. Knochentransportsystem (35) nach Anspruch 2, bei dem der Getriebemechanismus mindestens ein irreversibles kinematisches Paar aufweist.

4. Knochentransportsystem (35) nach Anspruch 1, bei dem der Schneckengetriebemechanismus (4) Folgendes aufweist:
eine Schnecke (5; 105), die drehbar am Hauptkörper (2; 102) befestigt ist;
und ein Schneckenrad (6; 106), das drehbar am Hauptkörper (2; 102) befestigt und mit der Schnecke (5; 105) in Eingriff ist;
wobei sich die Spule (8; 108) einheitlich mit Schneckenrad (6; 106) dreht.

5. Knochentransportsystem (35) nach Anspruch 4, wobei es ferner einen Knauf (12; 112) zum Übertragen einer Drehbewegung auf die Schnecke (5; 105) aufweist.

6. Knochentransportsystem (35) nach Anspruch 5, bei dem ferner der Knauf (12; 112) einen Arretiermechanismus aufweist, der die Drehung des Knaufs (12; 112) relativ zum Hauptkörper (2; 102) in bestimmten Arretierwinkelstellungen des Knaufs (12; 112) sperrt.

7. Knochentransportsystem (35) nach Anspruch 6, bei dem die Arretierwinkelstellungen festen Winkelinkrementen in der Drehung des Knaufs (12; 112) entsprechen.

8. Knochentransportsystem (35) nach Anspruch 7, bei dem der Arretiermechanismus einen als regelmäßiges Polygon ausgebildeten Vorsprung (24) und eine damit zusammenpassende Ausnehmung (25) aufweist, der bzw. die am Knauf (12) bzw. am Hauptkörper (2), oder andersherum, vorgesehen sind, wobei die Drehung der Schnecke (5) verhindert wird, wenn der als regelmäßiges Polygon ausgebildete Vorsprung (24) in die Ausnehmung (25) gedrückt wird, wobei der Arretiermechanismus ferner ein elastisches Element (22) aufweist, das den Knauf (12) in Richtung des Hauptkörpers (2) vorspannt.

9. Knochentransportsystem (35) nach Anspruch 4, und ferner mit einem mit der Schnecke (5; 105) verbundenen Motor, um die Schnecke (5; 105) drehanzutreiben, und einer Steuerung zum Steuern des Motors, wobei die Steuerung so konfiguriert ist, dass sie eine Aktivierungsfrequenz des Motors und einen Drehwinkel der von dem Motor angetriebenen Schnecke (5; 105) für jede Aktivierung des Motors steuert.

10. Knochentransportsystem (35) nach Anspruch 1, bei dem die Spule (8; 108) eine spiralförmige Rille (36) auf ihren Außenflächen hat, um das Kabel (9; 109) beim Aufwickeln aufzunehmen und dabei ein Überlappen zu verhindern.

11. Knochentransportsystem (35) nach Anspruch 1, und ferner mit einem direkt oder indirekt mit dem Kabel (9; 109) verbundenen Kraftsensor zum Erfassen einer auf das Kabel (9; 109) wirkenden Spannkraft.

12. Knochentransportsystem (35) nach Anspruch 1, bei dem das Element zum Umlenken des Kabels (9; 109) ein Drehpunktstift oder eine Drehpunktschraube (43; 143a; 143b) ist.

13. Knochentransportsystem (35) nach Anspruch 12, mit zwei Kabelziehvorrichtungen (1; 103) die fest an dem Fixateur externe (47) in diametral entgegengesetzten Positionen angebracht sind, wobei die beiden Kabelziehvorrichtungen (1; 103) die entgegengesetzten Enden desselben Kabels (9; 109) halten.

## Revendications

1. Système de transport osseux (35), comprenant:
un cadre de fixation externe (47) configuré pour être fixé à au moins un segment osseux fixe (40; 140); et
un élément pour rediriger le câble (9; 109) configuré pour être attaché au segment osseux fixe (40; 140) et pour rediriger le câble (9; 109) partant des dispositifs de traction de câble (1; 103) vers un segment de transport osseux (10; 110);
**caractérisé en ce qu'**il comprend en outre au moins un dispositif de traction de câble (1; 103) solidement attaché au cadre de fixation externe (47) comprenant:
un corps principal (2; 102) fixé au cadre de fixation externe (47) solidement attaché à au moins un segment osseux fixe (40; 140);
une bobine (8; 108) pouvant tourner autour dudit corps principal (2; 102), pour enrouler un câble (9; 109) destiné à être fixé à un segment osseux de transport (10; 110), déplaçant ainsi ledit segment osseux de transport (10; 110) vers ledit segment osseux fixe (40; 140);
un mécanisme de transmission entraînant la bobine (8, 108) afin d'enrouler le câble (9; 109).

2. Système de transport osseux (35) selon la revendication 1, dans lequel le mécanisme de transmission est un mécanisme d'engrenage à vis sans fin (4).

3. Système de transport osseux (35) selon la revendication 2, dans lequel le mécanisme de transmission comprend au moins une paire cinématique irréversible.

4. Système de transport osseux (35) selon la revendication 1, dans lequel le mécanisme d'engrenage à vis sans fin (4) comprend:
une vis sans fin (5; 105) fixé de manière rotative au corps principal (2; 102);
et une roue d'engrenage à vis sans fin (6; 106) fixée de manière rotative au corps principal (2; 102) et en prise avec ladite vis sans fin (5; 105);
ladite bobine (8; 108) tournant solidairement avec ladite roue d'engrenage à vis sans fin (6; 106).

5. Système de transport osseux (35) selon la revendication 4, dans lequel il comprend en outre un bouton (12; 112) pour transmettre un mouvement rotatif à ladite vis sans fin (5; 105).

6. Système de transport osseux (35) selon la revendication 5, en outre dans lequel ledit bouton (12; 112) comporte un mécanisme de blocage qui bloque la rotation du bouton (12; 112) par rapport au corps principal (2; 102) dans des positions angulaires de blocage prédéterminées du bouton (12; 112).

7. Système de transport osseux (35) selon la revendication 6, dans lequel les positions angulaires de blocage correspondent à des incréments angulaires fixes dans la rotation du bouton (12; 112).

8. Système de transport osseux (35) selon la revendication 7, dans lequel le mécanisme de blocage comprend un bossage polygonal régulier (24) et un évidement correspondant (25) prévus respectivement sur le bouton (12) et sur le corps principal (2), ou inversement, dans lequel la rotation de la vis sans fin (5) est empêchée lorsque le bossage polygonal régulier (24) est enfoncé dans l'évidement (25), le mécanisme de blocage comprenant en outre un élément élastique (22) qui sollicite le bouton (12) vers le corps principal (2).

9. Système de transport osseux (35) selon la revendication 4, comprenant en outre un moteur couplé à la vis sans fin (5; 105) pour entraîner la rotation de la vis sans fin (5; 105) et un contrôleur pour commander le moteur, dans lequel le contrôleur est configuré pour contrôler une fréquence d'activation du moteur et un angle de rotation de la vis sans fin (5; 105) entraînée par le moteur pour chaque activation du moteur.

10. Système de transport osseux (35) selon la revendication 1, dans lequel la bobine (8; 108) comprend une rainure hélicoïdale (36) sur ses surfaces externes pour recevoir le câble (9 ; 109) lorsqu'il s'enroule, empêchant ainsi tout chevauchement.

11. Système de transport osseux (35) selon la revendication 1, comprenant en outre un capteur de force couplé directement ou indirectement au câble (9; 109) pour détecter une force de tension agissant sur le câble (9; 109).

12. Système de transport osseux (35) selon la revendication 1, dans lequel ledit élément pour rediriger le câble (9; 109) est une broche ou une vis de renvoi (43; 143a; 143b).

13. Système de transport osseux (35) selon la revendication 12, comprenant deux dispositifs de traction de câbles (1; 103) solidement attachés au cadre de fixation externe (47) dans des positions diamétralement opposées, dans lequel les deux dispositifs de traction de câble (1; 103) maintiennent les extrémités opposées d'un même câble (9; 109).
